# EUROPÄISCHE PATENTSCHRIFT

(11) **EP 1 267 828 B1**
(45) Veröffentlichungstag und Bekanntmachung des Hinweises auf die Patenterteilung: **02.08.2006**
(21) Anmeldenummer: 01923641.3
(22) Anmeldetag: 06.03.2001
(51) Int. Cl.: A61K 9/16

(54) **PHARMAZEUTISCHE FORMULIERUNGEN ENTHALTEND SACCHAROSEFETTSÄUREESTER ZUR STEUERUNG DER WIRKSTOFFFREISETZUNG**
PHARMACEUTICAL PREPARATIONS CONTAINING SACCHAROSE FATTY ACID ESTERS FOR CONTROLLING THE RELEASE OF ACTIVE INGREDIENTS
FORMULATIONS PHARMACEUTIQUES COMPRENANT DES ESTERS D'ACIDES GRAS ET DE SACCHAROSE POUR CONTRÔLER LA LIBERATION DES AGENTS ACTIFS

(30) Priorität: 08.03.2000 DE 10010509; 09.03.2000 DE 60187962
(43) Veröffentlichungstag der Anmeldung: 02.01.2003
(73) Patentinhaber: AWD.pharma GmbH & Co.KG, 01097 Dresden (DE)
(72) Erfinder: HOFFMANN, Torsten, 01445 Radebeul (DE); PIEROTH, Michael, 01689 Weinböhla (DE); ZESSIN, Gerhard, 06114 Halle/Saale (DE); LANDGRAF, Karl-Friedrich, 01217 Dresden (DE)
(74) Vertreter: Uhlemann, Henry
(86) Internationale Anmeldenummer: PCT/EP2001/002500
(87) Internationale Veröffentlichungsnummer: WO 2001/066081

(56) Entgegenhaltungen:
- WO-A-00/13711
- WO-A-00/57853
- WO-A-99/49863
- NTAWUKULILYAYO J D ET AL: "Microcrystalline cellulose-sucrose esters as tablet matrix forming agents." INTERNATIONAL JOURNAL OF PHARMACEUTICS (AMSTERDAM), Bd. 121, Nr. 2, 1995, Seiten 205-210, XP001021333 ISSN: 0378-5173
- OBIKILI A ET AL: "IMPROVEMENT OF AQUEOUS SOLUBILITY AND DISSOLUTION KINETICS OF CANRENONE BY SOLID DISPERSION IN SUCRO ESTER" DRUG DEVELOPMENT AND INDUSTRIAL PHARMACY, Bd. 14, Nr. 6, 1988, Seiten 791-804, XP001017818 ISSN: 0363-9045
- PATENT ABSTRACTS OF JAPAN vol. 1996, no. 02, 29. Februar 1996 (1996-02-29) -& JP 07 267850 A (EISAI CO LTD), 17. Oktober 1995 (1995-10-17)

## Beschreibung

Die vorliegende Erfindung betrifft neue orale pharmazeutische Formulierungen mit variabel einstellbarem Freisetzungsverhalten des Wirkstoffes in Form von Granulaten, Pellets, Tabletten, Filmtabletten, Mikrotabletten, Dragees , Kapseln oder therapeutischen Systemen sowie Verfahren zu ihrer Herstellung durch Schmelzgranulation oder Schmelzpelletierung .

Bei der Verwendung von Arzneimitteln spielt die reduzierte Einnahmehäufigkeit, im Idealfall die tägliche Einmalgabe, eine bedeutende Rolle.

Eine Tablette morgens oder abends wird regelmäßiger eingenommen als mehrere Tabletten über den Tag verteilt. Diese erhöhte Patientenkompliance wirkt sich positiv auf den Heilungsprozeß aus. Zusätzlich kommt die häufig mit der verringerten Einnahmehäufigkeit einhergehende bessere Verträglichkeit des Wirkstoffes dem Patienten zugute. Letzteres hängt mit den dann erforderlichen längeren Erhalt der wirksamen Plasmakonzentration und meist auch gleichmäßigeren Plasmaspiegeln zusammen, bei denen unverträgliche Spitzenwerte weitgehend vermieden werden.

In Ausnahmefällen läßt sich bereits durch die kinetischen oder dynamischen Eigenschaften eines Wirkstoffes wie beispielsweise durch eine lange Eliminationshalbwertszeit eine Einmalgabe realisieren. In den allermeisten Fällen werden jedoch erst durch galenisch-technologische Maßnahmen, wie zum Beispiel durch eine verzögerte Wirkstofffreisetzung aus der Darreichungsform, wirksame Plasmaspiegel über 12 bis 24 Stunden ermöglicht.

In der Literatur gibt es dazu eine ganze Reihe von Prinziplösungen, die in Abhängigkeit von den chemischen und physikalischen Eigenschaften des Wirkstoffes Vor- beziehungsweise Nachteile zeigen (Übersichtsartikel: Recent trends and progress in sustained or controlled oral delivery of some water soluble drugs, Drug Development and Industrial Pharmacy 21 (9), 1037 -1070 (1998)).
Der Stand der Technik wird beispielsweise auch in einem der neueren Lehrbücher der pharmazeutischen Technologie wiedergegeben (Voigt, R., Pharmazeutische Technologie, Ullstein Mosby Verlag 1993, S 293 ff.). Danach kann die Wirkung von Arzneistoffen durch die folgenden Maßnahmen verlängert werden:
Molekülvariationen wie Ester- oder Salzbildungen, Veränderungen der Wirkstoffmodifikation, der Teilchengröße, Wahl entsprechender Hilfsstoffe und Verfahren. Beispielhaft soll auf einzelne Möglichkeiten kurz eingegangen werden:

### Matrix-Retardarzneiformen

Sie sind durch ein unlösliches, eventuell poröses Gerüst aus unverdaulichen Fetten, Wachsen, Polymeren oder auch anorganischen Matrixbildnem charakterisiert. In dieses Gerüst wird der Wirkstoff eingearbeitet. Die Wirkstofffreigabe erfolgt durch Diffusion, Erosion oder Matrixabbau.

### Hydrokolloid- Retardarzneiformen

Hierbei wird der Arzneistoff in Hydrokolloidmatrices eingebettet, die beispielsweise aus Cellulosederivaten bestehen. Nach Einnahme bildet sich durch die Verdauungsflüssigkeiten ein Gel, durch welches der Wirkstoff in Abhängigkeit von Oberfläche und Gelviskosität mehr oder weniger schnell diffundiert.

### Überzogene (membrankontrollierte) Retardarzneiformen

Wirkstoffpartikel oder Arzneiformen werden hierbei von einer Barriere umhüllt. Die Diffusion durch die Diffusionsbarriere bestimmt, wie schnell die

Wirkstofffreisetzung erfolgt. Zur Erhöhung der Diffusionsgeschwindigkeit können Weichmacher oder Porenbildner zugesetzt werden.

### Einfluß der spezifischen Oberfläche

Bei schlecht wasserlöslichen Wirkstoffen besteht im allgemeinen eine deutliche Abhängigkeit zwischen Auflösungsgeschwindigkeit und spezifischer Oberfläche. Durch gezielte Kristallisation des Wirkstoffes, durch Sieben oder Mahlen kann eine definierte Kornverteilung und damit eine bestimmte spezifische Oberfläche eingestellt werden. Je größer die Partikel sind, desto kleiner ist die spezifische Oberfläche und desto langsamer ist die Wirkstofffreisetzung.

### Mischformen aus Diffusion, Erosion, Lösevorgängen

Des weiteren sind Arzneiformen bekannt, deren verzögerte Wirkstofffreisetzung auf Kombination aus Diffusion, Erosion und Lösevorgängen beruhen.

Ein besonders interessantes und im Hinblick auf die Wirkstofffreisetzung sehr variabel einsetzbares Verfahren stellt die Schmelzgranulation dar.
Unter Schmelzgranulation oder thermoplastischer Granulation ist ein Prozeß zu verstehen, bei dem die Granulatbindung durch die Verwendung eines niedrig schmelzenden Bestandteils sowie unter dem Einfluß von thermischer Energie durchgeführt wird. (Lüdemann, J.; APV- Kurs 231 vom 17.-18-06.1996)

Es werden hierbei zwei Subtypen unterschieden.
Beim Subtyp Feuchtgranulation ist die Prozeßtemperatur größer als der Schmelzpunkt des bindenden Bestandteils. Dieser liegt bei der Granulation als flüssiger oder halbfester Bestandteil vor. An die Stelle der Trocknung tritt bei der Schmelzgranulation das Abkühlen.

Vom Subtyp Sintergranulation spricht man, wenn die Prozeßtemperatur nicht den Schmelzpunkt des bindenden Bestandteils erreicht. Hierbei findet nur ein lokales Schmelzen an der Partikeloberfläche statt, so daß die Oberflächen ineinander diffundieren (Voigt, R.; Lehrbuch der pharmazeutischen Technologie; Verlag Chemie, S. 159 (1984)).

Bei dem niedrigschrnelzenden Bestandteil kann es sich um einen wirksamen Bestandteil oder einen Hilfsstoff handeln. Die Schmelzpunkte der Substanzen liegen aus Stabilitätsgründen in der Regel über 35°C. Am häufigsten werden Stoffe mit Schmelzpunkten im Bereich 50 - 90°C verwendet.
Bekannte Wirkstoffe als schmelzbare Substanzen sind Phenylsalicylat, Ibuprofen, α- Liponsäure und Meprobamat.
Als schmelzbare Hilfsstoffe werden wasserlösliche, quellbare und lipophile Substanzen eingesetzt. Als hydrophile zum Beispiel Macrogol, Polyvidon und Polymethacrylsäure-Derivate.
Kohlenwasserstoffe (Paraffin), Wachse, Fette und Fettsäuren sind Beispiele für eingesetzte lipophile Hilfsstoffe.
(Flanders, P.; Dyer, G.A.; Jordan, D.; Drug Dev. Ind. Pharm. 13 (&), 1001 - 1022 (1987); Schaefer, T.; Holm, P.; Kristensen, H.G.; Drug Dev. Ind. Pharm. 16, 1249 - 1277 (1990); McTaggart, C.M. et. al.; Int. J. Pharm. 19, 139 - 148 (1984); Kinget, R.; Kemel, R.; Acta Pharm. Technol. 31, 57 (1985))

Die Schmelzgranulation wird in der Regel in Wirbelschichtgranulatoren, Zentrifugalwirbelschichtgeräten oder schnelllaufenden Intensivmischern durchgeführt. Besonders der Einsatz von letzteren hat verfahrenstechnische Vorteile, da eine kostenintensive Luftaufbereitung entfallen kann. Gegenüber konventionellen Granuliermethoden mit organischen Lösungsmitteln entfallen dabei Aufwendungen für Ex- Schutz und Lösungsmittelrückgewinnung. Auch Restlösungsmittel im Produkt entfallen. Gegenüber der wäßrigen Granulation entfallen energieaufwendige Trocknungsprozesse. Hierbei wird die Verwendung von sogenannten Eintopfsystemen favorisiert.

Allgemein kann der Prozeß zur Schmelzgranulation wie folgt dargestellt werden:

| | |
|---|---|
| Mischen | Mischen |
| ↓ | ↓ |
| Bindemittelzugabe (fester Aggregatzustand) | Aufheizen |
| ↓ | ↓ |
| Aufheizen | Bindemittelzugabe (flüssiger Aggregatzustand) |
| ↓ | ↓ |
| Granulieren | Granulieren |
| ↓ | ↓ |
| Abkühlen | Abkühlen |
| ↓ | ↓ |
| ggf. Klassieren | ggf. Klassieren |

Die Zugabe des schmelzbaren Bindemittels kann fest oder flüssig, das heißt im geschmolzenen Zustand, erfolgen.

Bei fester Zugabe wird der schmelzbare Stoff während des Prozesses zum Schmelzen gebracht, deshalb wird diese Methode auch Aufschmelzmethode genannt.

Bei letzterer Methode werden entweder die festen Bestandteile vorgelegt, zu denen das flüssige Bindemittel zugegeben wird oder entsprechend der sogenannten Fusionsmethode, es wird das flüssige Bindemittel vorgelegt und die Feststoffe eingerührt. Dazu erfolgt das Aufheizen vor der Bindemittelzugabe.

Die Energiezuführung kann bei Intensivmischern auf verschiedenen Wegen erfolgen:
- mechanische Energie durch Mischwerkzeuge und Zerhacker
- Kontaktwärme über Mantel
- Strahlungsenergie durch IR oder Mikrowelle
- Warmlufteintrag in Produktbett

Auch aus der Patentliteratur sind eine Vielzahl von Verfahren zur Herstellung derartiger Formulierungen bekannt.
Formulierungen mit kontrollierter Freisetzung, die auf dem Wege der Schmelzgranulation hergestellt werden können, werden beispielsweise beschrieben in DE 24 26 812, EP 351 580, EP 654 263, EP 672 416, EP 729 751 und WO 93/18753.

Ntawukulilyayo et al, Journal of Pharmaceutics, Bd. 121, Nr.2, 1995 offenbart Tabletten, die durch direkte Kompression oder Feuchtgranulation hergestellt werden. Beschrieben wird ein Matrixsystem aus mikrokristalliner Zellulose und Sucrosepalmitat oder - stearat.

Nach Obikili et al, Improvement of aqueous solubility and dissolution kinetics of Canrenone, Bd. 14, 1988 soll die Löslichkeit des sehr schlecht wasserlöslichen Wirkstoffes Canrenone in Wasser durch die Herstellung einer festen Dispersion mit Sucroseester (Sucrosedistearat) verbessert werden. Die feste Dispersion wird erhalten durch Mischen der beiden Komponenten, ein vollständiges Schmelzen und schnelles Abkühlen. Um eine flüssige transparente Schmelze zu erreichen, wird das Gemisch in einem Silikonölbad auf Temperaturen von 110 °C bis 166 °C erhitzt. Bei diesen hohen Temperaturen karamellisiert der Sucroseester und führt zu einem bräunlichen glasigen Produkt.

In WO 99/49863 A1 sind Formulierungen mit dem Wirkstoff FK506 (Tacrolimus) und einem Sucrosefettsäurester beschrieben. Der Sucrosefettsäureester wird stets zu dem in Ethanol/Acteon gelösten Wirkstoff gegeben und in dem heißen organischen Lösungsmittel gelöst und die Lösung abgekühlt. Diese Verfahrensweise hat zur Folge, dass nach dem Abkühlen der Wirkstoff FK506 amorph vorliegt, d. h. einzelne Moleküle des Wirkstoff im Sucrosefettsäureester fein verteilt sind. Eine derartige amorphe Wirkstoffformulierung bewirkt, dass der Wirkstoff sich sehr schnell löst, da er feinverteilt vorliegt, und nicht etwa kristallin.

JP 07-26780 A offenbart pharmazeutische Formulierungen, die den unangenehmen Geschmack eines Wirkstoffes kaschieren sollen. Diese Formulierungen bestehen aus einem Cellulosederivat, Polyvinxlpyrrolidon, Gelatine, Carrageein oder Casein als wasserlöslichem Polymer und einer wachsartigen Substanz.

WO 00/57853 beschreibt pharmazeutische Formulierungen, erhältlich durch Mischen mindestens eines Wirkstoffes mit mindestens einem Extrusionszusatzstoff aus der Gruppe der mit Fettsäuren veresterten Polyalkohole, u. a. Sucrosefettsäureester, und der Hilfsstoffe Polyvinylpyrrolidon (PVP) oder Polyethylenglykol (PEG) und gemeinsamer Schmelzextrusion ohne Wärmezufuhr.

WO 00/13711 betrifft Calciumvalproat enthaltende pharmazeutische Zusammensetzungen mit retardierender Wirkstofffreisetzung. Diese Zusammensetzungen enthalten neben dem Wirkstoff Calciumvalproat ein Acrylpoymer und dem Zuckerester Saccharosepalmitat oder - stearat. Der Zusatz des Acrylpolymers ist notwendig, um die retardierende Wirkung zu bekommen.

WO 93/18753 beschreibt ein Prozeß, bei dem zu hergestellten Pellets zu einem späteren Zeitpunkt der Herstellung wasserunlösliche, hydrophobe, wachsähnliche Substanzen bei einer Temperatur zugegeben werden, bei der diese Substanzen schmelzen und zum Coating der Pellets führen. Diesen Prozeß nennt man "hot melt coating".

Unter der Voraussetzung der Thermostabilität aller am Prozeß beteiligten Ausgangsstoffe unter den vorherrschenden Prozeßbedingungen ist die Schmelzgranulation eine interessante Alternative zu anderen Granulationsmethoden wie zum Beispiel dem Granulieren mit organischen Lösungsmitteln oder dem Granulieren mit Wasser.

Die Schmelzpelletierung stellt hierbei eine Sonderform der Prozeßführung dar, bei der Granulatpartikel weitgehend einheitlicher Größe und ausgerundeter Form gefertigt werden.

Trotz der Anzahl der bekannten schmelzbaren Hilfsstoffe sind nur wenige Hilfsstoffe mit abgestuftem HLB- Wert (Hydrophilic-Lipophilic-Balance-Wert) beschrieben, die sich für Schmelzgranulations- oder Schmelzpelletisierungsprozesse besonders eignen.

Vertreter der wenigen Hilfsstoffe mit abgestuftem HLB- Wert sind hydrierte Speisefette, die unter der Handelsbezeichnung Gelucire geführt werden oder die Sorbitolfettsäureester, die z.B. als Span bekannt sind. Auch diese jedoch decken nicht den breiten HLB- Bereich von 1 bis 16 ab.

Mit den klassischen schmelzbaren Hilfsstoffen können in der Regel Freisetzungsabstufungen nur über die Wahl des Retardierungsmittels beziehungsweise dessen Menge vorgenommen werden. Oftmals läßt sich ein Bindemittel nur in Kombination mit einem anderen schmelzbaren Bindemittel, wie Polyethylenglycol, verarbeiten, da seine Granulatbildungskapazität allein nicht ausreicht. Diese Bindemittel erfordern zudem den Zusatz von Gleit- oder Formtrennmitteln. Einige haben eine wachsartige Konsistenz.
Bei den bekannten Verfahren zur Schmelzgranulation müssen oftmals die entstandenen und erstarrten Granulate einer aufwendigen Siebung zur Zerkleinerung unterzogen werden.

Bei dem Prinzip der Retardierung mittels einer Umhüllung (Coating) wird aufgrund der teilweise spröden, aber auch der relativ dünnen Filmhüllen beim Verpressen oft eine Zerstörung der Filmhüllen beobachtet, es sei denn, daß mit einer relativ hohen Außenphase dem gegengesteuert wird. Bei Zerstörung der Filmhülle erhöht sich die Wirkstofffreisetzung der Tabletten. Das bedeutet, daß die Wirkstofffreisetzung dieser Tabletten meist preßkraftabhängig ist. Oftmals wird bei diesem Verfahren die Wirkstofffreisetzung über die aufgesprühte Menge während der Herstellung eingestellt.
In Abhängigkeit von der Filmbildung und der Porosität kann es während der Lagerung zu Veränderungen bei der Wirkstofffreisetzung kommen , beispielsweise durch Nachhärten.

Eine Aufgabe der vorliegenden Erfindung besteht deshalb darin, orale pharmazeutische Formulierungen mit variabel einstellbarem Freisetzungsverhalten, das heißt von schnell bis retardiert freisetzend bereitzustellen. Bei den modifiziert oder retardiert freisetzenden Arzneiformen sollen sowohl nicht zerfallende Arzneiformen (sogenannte "single units"), bevorzugt aber schnell zerfallende und modifiziert oder retardiert aus den Granulaten freisetzende Arzneiformen (sogenannte "multiple units") hergestellt werden können.

Eine weitere Aufgabe der vorliegenden Erfindung besteht darin, Verfahren zur Herstellung derartige Retardformulierungen insbesondere durch Schmelzgranulation oder Schmelzpelletierung bereitzustellen.

Erfindungsgemäß wird die Aufgabe gelöst durch pharmazeutische Formulierungen mit den Merkmalen nach Anspruch 1 sowie durch Verfahren zur Herstellung dieser pharmazeutischen Formulierungen gemäß den Ansprüchen 15 bis 20. Vorteilhafte Ausgestaltungen beinhalten die Ansprüche 2 bis 14.

Entsprechend der vorliegenden Erfindung werden neue orale pharmazeutische Formulierungen mit variabel einstellbarem Freisetzungsverhalten bereitgestellt, die neben einem oder mehreren Wirkstoffen ein oder mehrere Saccharosefettsäureester als alleiniges freisetzungssteuerndes Mittel enthalten.
Bei den neuen pharmazeutischen Formulierungen handelt es sich um schnellfreisetzende bis retardiert freisetzende Arzneiformen.

Die erfindungsgemäßen pharmazeutische Formulierungen können in Form von Granulaten, Pellets, Tabletten, Filmtabletten, Mikrotabletten, Dragees, Kapseln sowie als therapeutische Systeme verabreicht werden.

Überraschenderweise sind Saccharosefettsäureester in der Lage, die Wirkstofffreisetzung in der gewünschten Art zu steuern und darüber hinaus die technologischen Eigenschaften bei der Herstellung der erfindungsgemäßen Formulierungen durch Schmelzgranirlation oder Schmelzpelletierung zu verbessern.

Saccharosefettsäurester sind unter anderem auch dazu geeignet, den Wirkstoff ohne Zusatz anderer Hilfsstoffe zu granulieren. Dadurch ist eine Bruttomassen-reduzierung im Vergleich zu anderen Verfahren, in denen mehrere schmelzbare Retardierungs- beziehungsweise Bindemittel eingesetzt werden müssen, möglich.
Saccharosefettsäureester, besonders Stearate mit niedriegem HLB-Wert können gleichzeitig als Gleit- und Formtrennmittel verwendet werden.

Saccharosefettsäureester sind nichtionische Tenside, die aus Mono-, Di-, Tri- und Polyestern der Saccharose als hydrophilem Bestandteil und aus gesättigten oder ungesättigten Fettsäuren als lipophilen Bestandteil bestehen. Durch Variation des Veresterungsgrades und der Art der Fettsäuren lassen sich Saccharosefettsäureester mit unterschiedlichen HLB-Werten herstellen, die Einfluß auf die biopharmazeutischen Eigenschaften, insbesondere die Wirkstofffreisetzung, die Stabilität der hergestellten pharmazeutischen Formulierung sowie auf das technologische Verhalten haben.
Sie sind nicht toxisch, bioabbaubar, geschmacks- und geruchlos sowie lagerstabil .
Die Saccharosefettsäureester mit einem Schmelzpunkt von > 30°C liegen bei Raumtemperatur im festen Aggregatzustand vor und haben einen HLB- Wert von 1- 16.

Saccharosefettsäureester werden beispielsweise auch unter den Bezeichnungen Sugarester oder Sucroseester unter anderem von den Firmen Mitsubishi (Markenname Ryoto), Gattefosse oder Sisterna oder anderen vertrieben.

Saccharosefettsäureester sind in der Literatur bekannt, so werden diese gemäß US 4 844 067 zur Verbesserung der Oberfläche von Seidenfasern sowie in WO 93117667 als Geschmacksverbesserer in pharmazeutischen Zubereitungen eingesetzt.

Ihre Hauptanwendung liegt im Bereich der Lebensmittelindustrie. So werden Saccharosefettsäureester z. B. zur Verbesserung der Mischung von Kaugummimassen verwendet, gegen Entmischung und Denaturierung von Fertiggetränken, bei Zuckerraffination, bei Kondensmilch und Kaffeeweißern eingesetzt.

Bei der Herstellung von Weizenmehlprodukten werden Saccharosefettsäureester beispielsweise als Stabilisatoren, zur Verbesserung der Textur, zur Vermeidung von Anbackungen und Kleben verwendet. Bei Milchprodukten zur Stabilisierung von Emulsionen und Vermeidung vom Proteinabbau. Saccharosefettsäureester verbessern das Kristallisationsverhalten, sind wirkungsvolle Emulgatoren und erniedrigen die Viskosität bei der Herstellung von Fetten und Ölen.

In US 3 896 238, US 4 150 114 und US 4 046 886 wird die Verwendung von Saccharosefettsäureestern in Kombination mit Alkylsulfoxid oder Phosphoroxiden in pharmazeutischen Zusammensetzungen zur Verbesserung der Penetration der aktiven Substanz durch die Haut aufgezeigt. Als spezielle Saccharosefettsäureester werden beispielsweise genannt:
Saccharosemonooctanat, -monolaurat, -monopalmitat, -monostearat als auch die Di- und Triester dieser Verbindungen.
In JP 81 75 437 wird die Verwendung von Saccharosefettsäureestern mit einem HLB-Wert von 1 bis 5 als Grundlage für Suppositorien offenbart.

In WO 88/06880 wird der Einsatz von Saccharosefettsäureester in topischen Applikationen beansprucht, wobei Mischungen von Mono- und Dialkylsaccharoseester mit einem HLB-Wert von 8 bis 16 zur Verbesserung der Penetration durch die Haut eingesetzt werden.

Bevorzugt werden Saccharosecocoat, Saccharoserizinoleat, Saccharoselaurat und Saccharosestearat verwendet.

Saccharosefettsäureester werden auch insbesondere in kosmetischen Produkten verwendet (FR 2 421 605, JP 81 24 034, JP 81 55 306).

In DE 40 03 844 werden pharmazeutische Zusammensetzungen beschrieben, die neben dem Wirkstoff Cyclosporin einen Fettsäuresaccharidmonoester und ein Verdünnungsmittel oder Trägerstoff enthalten.
Diese Zusammensetzungen ermöglichen eine Reduzierung der zur Erzielung einer wirksamen Therapie erforderlichen Ciclosporin-Dosierungsspiegel und führen damit zur Verringerung von unerwünschten Nebenwirkungen. Als Fettsäuresaccharidmonoester sind hierbei besonders geeignet: C₆₋₁₄-Fettsäuredisaccharidmonoester und C₈₋₁₈₋Fettsäuretrisaccharidmonoester.

In WO 93/00093 wird eine neue Retardformulierung für Diltiazem in Form von Spheroiden beansprucht, die sich zusammensetzt aus dem Wirkstoff, einem Benetzungsmittel (wetting agent) und einer Polymerumhüllung zur Steuerung der Freisetzung. Als Benetzungsmittel werden Saccharosefettsäureester eingesetzt. Die eigentliche Retardierung erfolgt mittels eines Polymers. Dabei wird das Benetzungsmittel mit dem Wirkstoff mittels Extrusion oder mittels Granulation mit organischen Lösungsmitteln verarbeitet. Das Coating der Extrudate erfolgt mit herkömmlichen Polymeren.
Als Benetzungsmittel werden beispielsweise auch C₁₂-C₂₀-Fettsäureester der Saccharose oder Xylose genannt.

In DE 198 40 152 wird eine Retardformulierung beansprucht, welche Calciumvalproat, mindestens ein Acrylpolymer und mindestens ein Zuckerester enthält, wobei durch das eingesetzte Acrylpolymer die gewünschte Retardierung erzielt wird. Es wird aufgezeigt, daß der Zuckerester allein keine nennenswerte Retardierung bewirkt.

Die Eignung von Saccharosefettsäureestern in den erfindungsgemäßen pharmazeutischen Formulierungen als alleiniges freisetzungssteuemdes Mittel war um so überraschender, da diese Saccharosefettsäureester zum einen schon seit längerem bekannt sind und zum anderen sich damit orale pharmazeutische Formulierungen mit variabel einstellbarem Freisetzungsverhalten auf einfache Art herstellen lassen.

Die erfindungsgemäß verwendeten Saccharosefettsäureester sind Ester von Saccharose mit gesättigte oder ungesättigte Fettsäuren oder Gemische davon. Besonders geeignet sind C₁₂- 6₂₂-Fettsäuren.
Bevorzugt werden Saccharosestearate, Saccharosepalmitate, Saccharoselaurate, Saccharosebehenate und Saccharoseoleate mit einem HLB-Wert von 1-16 verwendet.
Der Schmelzpunkt oder Schmelzbereich der erfindungsgemäß verwendeten Saccharosefettsäureester liegt zwischen 30 - 200 °C.
Bevorzugt werden Saccharosefettsäureester mit einem Schmelzpunkt oder Schmelzbereich von 40 - 150 °C eingesetzt.

Ein wesentlicher Vorteil der vorliegenden Erfindung besteht darin, dass das gewünschte Freisetzungsverhalten der neuen pharmazeutischen Formulierungen über Typ und Anteil des oder der eingesetzten Saccharosefettsäureester beziehungsweise über die Verfahrensparameter des Herstellungsverfahrens gesteuert werden kann.

Saccharosefettsäureester mit niedrigem HLB-Wert werden bevorzugt für die Erlangung einer retardierten Freisetzung eingesetzt.
Saccharosefettsäureester mit hohem HLB-Wert eignen sich bevorzugt für ein schnelles oder modifiziertes Freisetzungsverhalten.

Die Saccharosefettsäureester können in den erfindungsgemäßen pharmazeutischen Formulierungen mit einem Anteil von 1 bis 95 Gew.-%, bezogen auf den zu granulierenden Anteil (Innenphase) in der Rezeptur vorhanden sein. Bevorzugt wird ein Anteil zwischen 5 und 50 Gew.-% eingesetzt. Neben Saccharosefettsäureester können in der Innenphase der Wirkstoff allein oder Gemische des Wirkstoffs mit einem oder mehreren pharmazeutisch gebräuchlichen Hilfsstoffen vorliegen.

Eine weitere Ausführungsform der Erfindung besteht darin, daß Granulate oder Pellets, die Saccharosefettsäureester im Granulat enthalten mit Saccharosefettsäureester umhüllt sein können.
Der Anteil an Saccharosefettsäureester in der Umhüllung beträgt 1 - 60 Gew.-% , bevorzugt 3 - 20 Gew.- % bezogen auf die umhüllte Arzneiform.

Die Saccharosefettsäureester können allein oder gegebenenfalls auch in Kombination mit anderen schmelzbaren Hilfsstoffen verwendet werden. Teilweise ist für den Prozeß der Zusatz von einem oder mehreren Hilfsstoffen, wie Weichmacher von Vorteil.

Die erfindungsgemäßen oralen pharmazeutischen Formulierungen können als Wirkstoffe sowohl gut wasserlösliche bis praktisch wasserunlösliche Verbindungen enthalten.

Wirkstoffe folgender Indikationsgruppen sind hierfür geeignet, wobei die Aufzählung nicht abschließend ist:
Analeptika/Antihypoxämika (wie Coffein), Analgetika/Antirheumatika (wie Diclofenac, Morphin, Tramadol, Tilidin, Flupirtin), Antiallergika (wie Azelastin, Pseudoephedrin), Antiarrhythmika , wie Chinidin, Disopyramid, Diltiazem, Verapamil), Antidementiva (Nootropika) (wie Piracetam, Nicergolin, Xantinonicotinat, Pentifyllin, Vincamin), Antidiabetika (wie Glibenclamid), Antiemetika/Antivertiginosa (wie Betahistindimesilat, Dimenhydrinat), Antiepileptika (wie Carbamazepin, Valproinsäure, Calciumvalproatdihydrat, Retigabin), Antihypertonika (wie Talinolol, Fosinopril, Doxazosin, Metoprolol, Nifedipin), Antihypotonika (wie Norfenefrin-HCl, Dihydroergotaminmesilat), Broncholytika/Antiasthmatika (wie Salbutamol, Terbutalinsulfat, Theophyllin), Diuretika (wie Furosemid, Piretanid), durchblutungsfördernde Mittel (wie Buflomedil, Naftidrofuryl, Pentoxifyllin), Koronarmittel (wie Glyceroltrinitrat, Isosorbidmononitrat, Isosorbiddinitrat, Molsidomin), Lipidsenker (wie Bezafibrat, Fenofibrat, Xantinol), Migränemittel (wie Sumatriptan), Muskelrelaxantia, Parkinsonmittel u. andere Mittel gegen extrapyramidale Störungen (wie Levodopa/Benserazid, Levodopa/Carbidopa), Psychopharmaka (wie Amitriptylin- HCl, Venlafaxin- HCl, Thioridazin- HCl, Lithiumcarbonat, Lithiumacetat); Thioctsäure oder R-Thioctsäure und ihre Salze, wie Dexlipotam.

Die erfindungsgemäßen pharmazeutischen Formulierungen enthalten vorzugsweise Flupirtin, Tramadol, Nifedipin, Carbamazepin, Calciumvalproat oder Retigabin.

Die erfindungsgemäßen pharmazeutischen Formulierungen können entsprechend der vorliegenden Erfindung durch Schmelzgranulation oder Schmelzpelletierung hergestellt werden.
Hierbei wird beispielsweise in einem schnellaufenden Mischer das Gemisch aus dem Wirkstoff und einem oder mehreren Saccharosefettsäureestern, gegebenenfalls mit den anderen Hilfsstoffen unter Rühren oder in der Wirbelschicht erwärmt. Die Erwärmung kann entweder mittels Heizmantel, mit Mikrowelle, Strahlungsenergie oder aber über den Energieeintrag des Rührers erfolgen.

Wenn die Schmelztemperatur des jeweilig verwendeten Saccharosefettsäureesters im Gemisch erreicht oder deren Oberfläche erweicht oder angeschmolzen wird, beginnt die Granulation. Aufgrund der einsetzenden Agglomeration und der damit verbundenen Erhöhung der Reibung steigt die Leistungsaufnahme des Rührmotors.
Der Granulationsabbruch erfolgt in der Regel, wenn die Leistungsaufnahme expotentiell beginnt zu steigen. Danach wird das warme Schmelzgranulat entweder aus dem Mischer ausgetragen und in dünnen Schichten bei Raumtemperatur abgekühlt oder aber mit geeigneter Kühlung (z.B. Kühlmantel) im Mischer eventuell unter Rühren abgekühlt. Erfindungsgemäß besteht auch die Möglichkeit der Zugabe der Saccharosefettsäureester im geschmolzenen Zustand.

Überraschenderweise wird dabei eine sehr enge Granulatgrößenverteilung erreicht. Die Granulat- beziehungsweise Pelletpartikel besitzen je nach Prozeßführung eine nahezu gerundete und glatte Oberfläche.

Ebenso ist die Verwendung anderer aufheizbarer Geräte, wie Wirbelschichtgranulator, Rotorgranulator möglich.

Die so hergestellten Granulate können gegebenenfalls über ein Sieb klassiert werden, eventuell mit Hilfsstoffen der Außenphase gemischt und beispielsweise zu Tabletten verpreßt oder in Kapseln abgefüllt werden.
Als Hilfsstoffe der Außenphase können pharmazeutisch übliche Sprengbeziehungsweise Zerfallshilfsmittel, Füllstoffe, Formtrennmittel oder ähnliches verwendet werden. In der Regel kann auf den Einsatz von Formtrennmitteln bei Verwendung von Saccharosestearaten niedrigen HLB- Werts verzichtet werden, da Saccharosestearate mit niedrigem HLB- Wert selbst auch gute Formtrennmittel darstellen.

Je nach dem pharmazeutisch-technologischen Ziel können so beispielsweise schnellfreisetzende oder modifiziert bis retardiert freisetzende Formulierungen, (multiple units oder single unit) hergestellt werden.

Des weiteren wurde überraschend gefunden, daß die Saccharosefettsäureester als Hilfsstoffe zum "hot melt coating" geeignet sind.
Dazu wird zu einem bereits hergestellten und erstarrten Schmelzgranulat nochmals eine Menge Saccharosefettsäureester gleichen oder abweichenden Typs zugegeben und die Mischung nochmals über den Schmelzpunkt beziehungsweise die Erweichungstemperatur des zugegebenen Saccharosefettsäureesters erwärmt. Dabei erfolgt ein Überzug der Schmelze des Saccharosefettsäureesters über das Schmelzgranulat.
Das Coating kann ebenso im Beisein eines Weichmachers erfolgen.
Ebenso können saccharosefettsäureesterfreie Granulate oder reine Wirkstoffe in der beschriebenen Art und Weise überzogen werden.

Die Vorteile dieses Verfahrens liegen darin, daß zum einen durch den Überzug eine ausreichende Freisetzungssteuerung, besonders Retardierung bereits mit geringeren Anteilen an Saccharosefettsäureestern erreicht wird. Zum anderen wird die Oberfläche der so hergestellten Granulate oder Pellets geglättet.

Ein weiterer Vorteil besteht darin, dass mit diesem Verfahren auf einfache Weise magensaftresistente Überzüge herstellbar sind. Damit besteht die Möglichkeit, die Wirkstofffreisetzung im sauren pH-Bereich stark zu verzögern aufgrund der praktischen Unlöslichkeit der Saccharosefettsäureester in wäßrigen und sauren Medien.

Das "powder coating" stellt hierbei eine besondere Form des "hot melt coatings" dar. Hierbei werden zum einen die gut rieselfähigen Saccharosefettsäureester mit Hilfe einer geeigneten Pulverzuführung, wie powder feeder, und andererseits ein Weichmacher wie Triethylcitrat zu den vorgelegten Ausgangsstoffen zudosiert
Dieses Verfahren zeichnet sich durch hohe Kosten- und Zeitersparnis aus, es sind gegenüber konventionellen wäßrigen Coating-Verfahren keine Trocknungsprozesse erforderlich.
Insbesondere eignen sich derartig hergestellte pharmazeutische Formulierungen für wasserempfindliche Wirkstoffe, wie Na-Valproat.

Die nachfolgenden Beispiele sollen den Gegenstand der Erfindung näher erläutern ohne diesen einzuschränken.

### Beispiel 1:

### Tramadolhydrochlorid mit 50% Saccharosestearat HLB-Wert 1

- Rezeptur

| Substanz | Einwaage |
|---|---|
| Tramadolhydrochlorid | 400g |
| Saccharosestearat S- 170 | 400g |

- Parameter:

| | | |
|---|---|---|
| Ansatzmenge | 800 g | |
| Rührerdrehzahl | 700 U/min | |
| Zerhackerdrehzahl | 3000 U/min | |
| Manteltemperatur | 55,0°C | |

Die Ausgangsstoffe werden in einem Intensivmischer vom Typ GP1 der Fa. Aeromatic- Fielder bei der entsprechenden Manteltemperatur unter Rühren erwärmt. Bei Erreichen einer bestimmten Produkttemperatur beginnt der Granulationsprozeß. Nach Erreichen eines Anstiegs der Leistungsaufnahme des Rührmotors und einem plötzlichen Produkttemperaturanstieg wird die Granulation abgebrochen und das Produkt ausgetragen, über ein Sieb der Maschenweite 1,4 mm gegeben und bei Raumtemperatur abgekühlt.
- Auswertung: Wirkstofffreisetzung

| Zeit in min | 30 | 60 | 120 | 180 | 240 | 360 | 480 |
|---|---|---|---|---|---|---|---|
| Freisetzung in % in 0,1 HCl | 74,03 | 89,40 | 95,75 | 95,57 | 97,61 | 98,58 | 97,87 |
| In Puffer pH 6,8 | 78,99 | 89,29 | 93,99 | 93,37 | 94,26 | 96,5 | 96,88 |

Wirkstofffreisetzung siehe Anlage 1

### Beispiel 2:

### Flupirtinmaleat mit 30% Saccharosestearat HLB-Wert 1

- Rezeptur:

| Substanz | Einwaage |
|---|---|
| Flupirtinmaleat | 240,0 g |
| Saccharosestearat S-170 | 102,9 g |

- Parameter:

| | | |
|---|---|---|
| Ansatzmenge | 342,9 g | |
| Rührerdrehzahl | 700 U/min | |
| Zerhackerdrehzahl | 3000 U/min | |
| Manteltemperatur | 61,2°C | |

- Herstellung erfolgt gemäß Beispiel 1.

### Beispiel 3:

### Nifedipin mit 30% Saccharosestearat HLB-Wert 1

- Rezeptur:

| Substanz | Einwaage |
|---|---|
| Nifedipin | 560 g |
| Saccharosestearat S- 170 | 240 g |

- Parameter:

| | | |
|---|---|---|
| Ansatzmenge | 800 g | |
| Rührerdrehzahl | 700 U/min | |
| Zerhackerdrehzahl | 3000 U/min | |
| Manteltemperatur | 58°C | |

- Herstellung erfolgt gemäß Beispiel 1.
- Auswertung: Wirkstofffreisetzung

| Zeit in h | | 1 | 2 | 4 | 6 | 8 | 24 |
|---|---|---|---|---|---|---|---|
| Freisetzung in % in gereinigtem Wasser/1,25% SDS | | 2,14 | 3,76 | 5,84 | 8,42 | 10,72 | 25,91 |

Wirkstofffreisetzung siehe Anlage 2

### Beispiel 4:

### Nifedipin mit 30% Saccharosepalmitat HLB-Wert 1

- Rezeptur:

| Substanz | Einwaage |
|---|---|
| Nifedipin | 560 g |
| Saccharosepalmitat P-170 | 240 g |

- Parameter:

| | | |
|---|---|---|
| Ansatzmenge | 800 g | |
| Rührerdrehzahl | 700 U/min | |
| Zerhackerdrehzahl | 3000 U/min | |
| Manteltemperatur. | 52°C | |

- Herstellung erfolgt gemäß Beispiel 1.
- Auswertung: Wirkstofffreisetzung

| Zeit in h | | 1 | 2 | 4 | 6 | 8 | 24 |
|---|---|---|---|---|---|---|---|
| Freisetzung in % in gereinigtem Wasser/1,25% SDS | | 4,08 | 7,32 | 11,5 | 16,65 | 21,71 | 49,04 |

Wirkstofffreisetzung siehe Anlage 3

### Beispiel 5:

### Tabletten aus Schmelzgranulat von Nifedipin mit 30% Saccharosestearat HLB-Wert 5

- Rezeptur:

| Substanz | Einwaage |
|---|---|
| Nifedipin | 560 g |
| Saccharosestearat S-570 | 240 g |

- Parameter für Granulierung:

| | | |
|---|---|---|
| Ansatzmenge | 800 g | |
| Rührerdrehzahl | 700 U/min | |
| Zerhackerdrehzahl | 3000 U/min | |
| Manteltemperatur | 70°C | |

- Herstellung erfolgt gemäß Beispiel 1.
- Parameter für Tablettierung :

Das Granulat wurde im Anschluß mit Tablettierwerkzeug rund, 6 mm Durchmesser, mittelgewölbt zu Tabletten mit einer Bruttomasse von 71,4 mg verpreßt.
- Auswertung: Wirkstofffreisetzung

| Zeit in min | 30 | 60 | 120 | 180 | 240 |
|---|---|---|---|---|---|
| Freisetzung in % in gereinigtem Wasser /1.25% SDS | 19,85 | 42,44 | 78,30 | 96,61 | 102,88 |

Wirkstofffreisetzung siehe Anlage 4

### Beispiel 6:

### Tabletten aus Schmelzgranulat von Nifedipin mit 50% Saccharosestearat HLB-Wert 9 und 2,5% Saccharosestearat HLB-Wert 1

- Rezeptur:

| Substanz | Einwaage |
|---|---|
| Nifedipin | 400 g |
| Saccharosestearat S- 970 | 380 g |
| Saccharosestearat S-170 | 20 g |

- Parameter für Granulierung:

| | | |
|---|---|---|
| Ansatzmenge | 800 g | |
| Rührerdrehzahl | 700 U/min | |
| Zerhackerdrehzahl | 3000 U/min | |
| Manteltemperatur | 65°C | |

- Die Herstellung erfolgt gemäß Beispiel 1
- Parameter für Tablettierung :
Das Granulat wurde mit Tablettierwerkzeug rund, 6mm Durchmesser, mittelgewölbt zu Tabletten mit einer Bruttomasse von 100 mg verpreßt.
- Auswertung Wirkstofffreisetzung:

| Zeit in min | 30 | 60 | 120 | 180 | 240 |
|---|---|---|---|---|---|
| Freisetzung in % in gereinigtem Wasser 12,5% SDS | 20,10 | 40,37 | 73,26 | 94,14 | 102,93 |

Wirkstofffreisetzung siehe Anlage 5

### Beispiel 7:

### Carbamazepin mit 10% Saccharosestearat HLB-Wert 1

- Rezeptur:

| Substanz | Einwaage |
|---|---|
| Carbamazepin | 720 g |
| Saccharosestearat S-170 | 80 g |

- Parameter:

| | | |
|---|---|---|
| Ansatzmenge | 800 g | |
| Rührerdrehzahl | 700 U/min | |
| Zerhackerdrehzahl | 3000 U/min | |
| Manteltemperatur | 55°C | |

- Die Herstellung erfolgt gemäß Beispiel 1
- Auswertung: Wirkstofffreisetzung

| Zeit in min | 30 | 60 | 120 | 180 | 240 | 360 | 480 |
|---|---|---|---|---|---|---|---|
| Freisetzung in % in modifiziertem Darmsaft | 10,68 | 20,06 | 38,08 | 51,45 | 62,47 | 73,89 | 81,58 |

Wirkstofffreisetzung siehe Anlage 6

### Beispiel 8:

### Carbamazepin mit 30% Saccharosestearat HLB-Wert 9

- Rezeptur:

| Substanz | Einwaage |
|---|---|
| Carbamazepin | 560 g |
| Saccharosestearat S- 970 | 240 g |

- Parameter:

| | | |
|---|---|---|
| Ansatzmenge | 800 g | |
| Rührerdrehzahl | 700 U/min | |
| Zerhackerdrehzahl | 3000 U/min | |
| Manteltemperatur | 68°C | |

- Die Herstellung erfolgt gemäß Beispiel 1
- Auswertung: Wirkstofffreisetzung

| Zeit in min | 30 | 60 | 120 | 180 | 240 | 360 | 480 |
|---|---|---|---|---|---|---|---|
| Freisetzung in % in modifiziertem Darmsaft | 26,09 | 42,27 | 62,65 | 80,58 | 87,38 | 96,56 | 100,84 |

Wirkstofffreisetzung siehe Anlage 7

### Beispiel 9:

### Carbamazepin mit 50% Saccharosebehenat HLB-Wert 3 und 2,5% Triethylcitrat

- Rezeptur:

| Substanz | Einwaage |
|---|---|
| Carbamazepin | 400 g |
| Saccharosebehenat B- 370 | 380 g |
| Triethylcitrat | 20 g |

- Parameter:

| | | |
|---|---|---|
| Ansatzmenge | 800 g | |
| Rührerdrehzahl | 700 Ulmin | |
| Zerhackerdrehzahl | 3000 U/min | |
| Manteltemperatur | 50°C | |

In einem Intensivmischer vom Typ GP1 der Fa. Aeromatic- Fielder werden die Ausgangsstoffe Carbamazepin und Triethylcitrat gemischt. Nach 1 min Mischzeit wird Saccharosebehenat B- 370 zugegeben und die Mischung bei einer Manteltemperatur von 50,0°C unter Rühren erwärmt. Nach Erreichen einer bestimmten Produkttemperatur, bei der ein Leistungsanstieg zu beobachten ist, wird das Granulat über ein Sieb der Maschenweite 1,4 mm gegeben und auf Raumtemperatur abgekühlt.

### Beispiel 10:

### Tabletten aus Schmelzgranulat von Carbamazepin mit 30% Saccharosestearat HLB-Wert 9

- Rezeptur:

| Substanz | Einwaage |
|---|---|
| Carbamazepin | 560 g |
| Saccharosestearat S-970 | 240 g |

- Parameter für Granulierung:

| | | |
|---|---|---|
| Ansatzmenge | 800 g | |
| Rührerdrehzahl | 700 U/min | |
| Zerhackerdrehzahl | 3000 U/min | |
| Manteltemperatur | 68°C | |

- Die Herstellung erfolgt gemäß Beispiel 1
- Parameter für Tablettierung:
Das Granulat wird ohne weitere Zusätze mit Tablettierwerkzeug rund, 13 mm Durchmesser, flach zu Tabletten mit einer Bruttomasse von 571 mg mit einer Druckfestigkeit von 25 N verpreßt.
- Auswertung: Wirkstofffreisetzung

| Zeit in min | 30 | 60 | 120 | 180 | 240 | 360 | 480 |
|---|---|---|---|---|---|---|---|
| Freisetzung in % in modifiziertem Darmsaft | 5,36 | 8,04 | 13,78 | 17,89 | 21,01 | 27,31 | 32,08 |

Wirkstofffreisetzung siehe Anlage 8

### Beispiel 11:

### Carbamazepin mit 20% Saccharosestearat HLB-Wert 2

- Rezeptur:

| Substanz | Einwaage |
|---|---|
| Carbamazepin | 640 g |
| Saccharosestearat S- 270 | 160 g |

- Parameter:

| | | |
|---|---|---|
| Ansatzmenge | 800 g | |
| Rührerdrehzahl | 700 U/min | |
| Zerhackerdrehzahl | 3000 U/min | |
| Manteltemperatur | 54°C | |

- Die Herstellung erfolgt gemäß Beispiel 1

### Beispiel 12:

### Calciumvalproatdihydrat mit 35% Calciumhydrogenphosphat und 30% Saccharosestearat HLB-Wert 1

- Rezeptur:

| Substanz | Einwaage |
|---|---|
| Calciumvalproatdihydrat | 280 g |
| Calciumhydrogenphosphat | 280 g |
| Saccharosestearat S-170 | 240 g |

- Parameter

| | | |
|---|---|---|
| Ansatzmenge | 800 g | |
| Rührerdrehzahl | 700 U/min | |
| Zerhackerdrehzahl | 3000 U/min | |
| Manteltemperatur | 53°C | |

- Die Herstellung erfolgt gemäß Beispiel 1, wobei der Wirkstoff
Calciumvalproatdihydrat mit Calciumhydrogenphosphat vorgelegt werden.
- Auswertung: Wirkstofffreisetzung

| Zeit in min | 60 | 240 | 480 |
|---|---|---|---|
| Freisetzung in % in pH 3,0 | 64,89 | 75,63 | 85,02 |
| Freisetzung in % in pH 6,8 | 36,85 | 61,26 | 71,60 |

Wirkstofffreisetzung siehe Anlage 9

### Beispiel 13:

### Tabletten aus Schmelzgranulat von Calciumvalproatdihydrat und 30% Saccharosestearat HLB-Wert 1

- Rezeptur:

| Substanz | Einwaage |
|---|---|
| Calciumbvalproatdihydrat | 560 g |
| Saccharosestearat S- 170 | 240 g |

- Parameter für Granulierung:

| | | |
|---|---|---|
| Ansatzmenge | 800 g | |
| Rührerdrehzahl | 700 U/min | |
| Zerhackerdrehzahl | 3000 U/min | |
| Manteltemperatur | 55°C | |

- Die Herstellung erfolgt gemäß Beispiel 1
- Parameter für Tablettierung:
Das Granulat wird mit Tablettienrverkzeug oblong, 23 mm lang, 9 mm breit zu Oblongtabletten mit einer Bruttomasse von 951 mg und einer Druckfestigkeit von 65 N verpreßt.
- Auswertung: Wirkstofffreisetzung

| Zeit in min | 60 | 240 | 480 |
|---|---|---|---|
| Freisetzung in % in pH 3,0 | 4,96 | 9,14 | 13,66 |
| Freisetzung in % in pH 6,8 | 92,93 | 98,57 | 99,43 |

Wirkstofffreisetzung siehe Anlage 10

### Beispiel 14:

### Tabletten aus Schmelzgranulat von Calciumvalproatdihydrat und 30% Saccharosestearat HLB-Wert 9

- Rezeptur:

| Substanz | Einwaage |
|---|---|
| Calciumvalproatdihydrat | 560 g |
| Saccharosestearat S- 970 | 240 g |

- Parameter für Granulierung:

| | | |
|---|---|---|
| Ansatzmenge | 800 g | |
| Rührerdrehzahl | 700 U/min | |
| Zerhackerdrehzahl | 3000 U/min | |
| Manteltemperatur | 65°C | |

- Die Herstellung erfolgt gemäß Beispiel 1
- Parameter für Tablettierung:
Das Granulat wird mit Tablettierwerkzeug oblong, 23 mm lang, 9 mm breit zu Oblongtabletten mit einer Bruttomasse von 951 mg und einer Druckfestigkeit von 50 N verpreßt.
- Auswertung: Wirkstofffreisetzung

| Zeit in min | 60 | 240 | 480 |
|---|---|---|---|
| Freisetzung in % in pH 3,0 | 16,89 | 56,55 | 87,96 |
| Freisetzung in % in pH 6,8 | 1,75 | 2,77 | 4,34 |

Wirkstofffreisetzung siehe Anlage 11 a
Vergleich der Wirkstofffreisetzung der Calciumvalproatrezepturen in pH 3,0 siehe Anlage 11 b
Vergleich der Wirkstofffreisetzung der Calciumvalproatrezepturen in pH 6,8 siehe Anlage 11 c

### Beispiel 15:

### Retigabin mit 20% Saccharosestearat HLB-Wert 1

- Rezeptur:

| Substanz | Einwaage |
|---|---|
| Retigabin | 800 g |
| Saccharosestearat S-170 | 200 g |

- Parameter

| | | |
|---|---|---|
| Ansatzmenge | 1000 g | |
| Rührerdrehzahl | 700 U/min | |
| Zerhackerdrehzahl | 3000 U/min | |
| Manteltemperatur | 52°C | |

- Die Herstellung erfolgt gemäß Beispiel 1
- Auswertung : Wirkstofffreisetzung

| Zeit in min | 30 | 60 | 120 | 180 | 240 | 360 | 480 |
|---|---|---|---|---|---|---|---|
| Freisetzung in % in 0,1N HCl | 37,23 | 56,71 | 75,81 | | | | |
| in Puffer pH 6.8/1% Texapon | 5,71 | 8,77 | 13,82 | 15,79 | 23,60 | 27,99 | 35,62 |

Wirkstofffreisetzung siehe Anlage 12

### Beispiel 16:

### Retigabin mit 20% Saccharosestearat HLB-Wert 2

- Rezeptur:

| Substanz | Einwaage |
|---|---|
| Retigabin | 400 g |
| Saccharosestearat S- 270 | 100 g |

- Parameter

| | | |
|---|---|---|
| Ansatzmenge | 500 g | |
| Rührerdrehzahl | 700 U/min | |
| Zerhackerdrehzahl | 3000 U/min | |
| Manteltemperatur | 55°C | |

- Die Herstellung erfolgt gemäß Beispiel 1
- Auswertung Wirkstofffreisetzung:

| Zeit in min | 15 | 30 | 60 | 120 | 180 | 240 | 360 | 480 |
|---|---|---|---|---|---|---|---|---|
| Freisetzung in % in 0,1 N HCl | 42,28 | 62,58 | 83,53 | 100,97 | | | | |
| Freisetzung in % in Puffer pH 7,5 | | 11,82 | 20,77 | 34,41 | 44,94 | 52,74 | 61,63 | 66,37 |

Wirkstofffreisetzung siehe Anlage 13

### Beispiel 17:

### Retigabin mit 20% Saccharosestearat HLB-Wert 1 und 10% Saccharosestearat HLB-Wert 9

- Rezeptur:

| Substanz | Einwaage |
|---|---|
| Retigabin | 210 g |
| Saccharosestearat S- 170 | 60 g |
| Saccharosestearat S- 970 | 30 g |

- Parameter

| | | |
|---|---|---|
| Ansatzmenge | 300 g | |
| Rührerdrehzahl | 500 U/min | |
| Zerhackerdrehzahl | 3000 U/min | |
| Manteltemperatur | 65°C | |

- Die Herstellung erfolgt gemäß Beispiel 1
- Auswertung Wirkstofffreisetzung:

| Zeit in min | 15 | 30 | 60 | 120 | 180 | 240 | 360 | 480 |
|---|---|---|---|---|---|---|---|---|
| Freisetzung in % in 0,1 N HCl | 71,49 | 85,13 | 97,93 | 102,82 | | | | |
| Freisetzung in % in Puffer 6,8 | | 31,02 | 36,93 | 51,97 | 61,25 | 70,63 | 79,01 | 78,77 |

Wirkstofffreisetzung siehe Anlagen 14 a
Vergleich der Wirkstofffreisetzungen von Retigabinrezepturen in 0,1 N HCl siehe Anlage 14 b
Vergleich der Wirkstofffreisetzungen von Retigabinrezepturen in Puffer pH 6,8 siehe Anlage 14 c

### Beispiel 18:

### Tabletten aus Schmelzqranulat mit Retiqabin, 20% Saccharosestearat HLB-Wert 1 und 10% Croscarmelose-Natrium

- Rezeptur

| Substanz | Einwaage |
|---|---|
| Retigabin | 800 g |
| Saccharosestearat S- 170 | 200 g |

- Parameter für Granulierung:

| | | |
|---|---|---|
| Ansatzmenge | 1000 g | |
| Rührerdrehzahl | 700 U/min | |
| Zerhackerdrehzahl | 3000 U/min | |
| Manteltemperatur | 52°C | |

- Die Herstellung erfolgt gemäß Beispiel 1
- Parameter für Tablettierung:

| Substanz | Einwaage |
|---|---|
| Retigabin retard - Granulat (s.o.) | 270 g |
| Croscarmellose-Natrium | 30 g |

Die Tablettiermischung wird mit Tablettierwerkzeug rund, Durchmesser 9mm, Fase 45°, Wölbungsradius R13 zu Tabletten verpreßt.
- Auswertung: Wirkstofffreisetzung

| Zeit in min | 15 | 30 | 60 | 120 | 180 | 240 | 360 | 480 |
|---|---|---|---|---|---|---|---|---|
| Freisetzung in % in 0,1N HCl | 40,76 | 81,59 | 96,13 | 100,76 | | | | |
| In Puffer pH 6,8 / 1%Texapon | | 22,20 | 29,80 | 38,95 | 46,49 | 53,58 | 60,85 | 64,69 |

Wirkstofffreisetzung siehe Anlage 15

### Beispiel 19:

### Retigabin mit 7% Saccharosestearat HLB-Wert 1

- Rezeptur:

| Substanz | Einwaage |
|---|---|
| Retigabin | 372 g |
| Saccharosestearat S- 170 | 28 g |

- Parameter

| | |
|---|---|
| Ansatzmenge | 400 g |
| Rührerdrehzahl | 1300 U/min |
| Manteltemperatur | 50°C |

In einem Intensivmischer vom Typ GP1 der Fa. Aeromatic- Fielder werden die Ausgangsstoffe in einem speziellen, mit einem PTFE- Inliner versehenen Behälter bei einer Manteltemperatur von 50,0°C unter Rühren erwärmt. Nach dem Wiederanstieg der Leistungsaufnahme werden die Pellets entnommen und in dünnen Schichten auf Raumtemperatur abgekühlt.
- Auswertung: Wirkstofffreisetzung

| Zeit in min | 30 | 60 | 120 | 180 | 240 | 360 | 480 |
|---|---|---|---|---|---|---|---|
| Freisetzung in Puffer PH 7,5/2,5%Texapon | 16,62 | 29,85 | 50,39 | 67,14 | 69,35 | 83,20 | 90,96 |

Wirkstofffreisetzung siehe Anlage 16

### Beispiel 20:

### Retigabin mit 20% Saccharosestearat HLB-Wert 11

- Rezeptur:

| Substanz | Einwaage |
|---|---|
| Retigabin | 320 g |
| Saccharosestearat S-1170 | 80 g |

- Parameter

| | |
|---|---|
| Ansatzmenge | 400 g |
| Rührerdrehzahl | 1300 - 1100 U/min |
| Manteltemperatur | 50°C |

- Herstellung erfolgt gemäß Beispiel 19
- Auswertung : Wirkstofffreisetzung

| Zeit in min | 30 | 60 | 120 | 180 | 240 |
|---|---|---|---|---|---|
| Freisetzung in Puffer PH 7,5/2,5%Texapon | 49,91 | 79,95 | 100,81 | 106,03 | 104,36 |

Wirkstofffreisetzung siehe Anlage 17

### Beispiel 21:

### Retigabin mit 20% Saccharosestearat HLB-Wert 16

- Rezeptur:

| Substanz | Einwaage |
|---|---|
| Retigabin | 320 g |
| Saccharosestearat S-1670 | 80 g |

- Parameter

| | |
|---|---|
| Ansatzmenge | 400 g |
| Rührerdrehzahl | 1300 - 1100 U/min |
| Manteltemperatur | 50- 55°C |

- Herstellung erfolgt gemäß Beispiel 19
- Auswertung : Wirkstofffreisetzung

| Zeit in min | 30 | 60 | 120 | 180 | 240 |
|---|---|---|---|---|---|
| Freisetzung in Puffer PH 7,5/2,5%Texapon | 41,77 | 68,71 | 92,32 | 99,95 | 101,47 |

Wirkstofffreisetzung siehe Anlage 18

### Beispiel 22:

### Retigabin mit 16% Saccharosestearat HLB-Wert 15

- Rezeptur:

| Substanz | Einwaage |
|---|---|
| Retigabin | 336 g |
| Saccharosestearat S-1570 | 64 g |

- Parameter:

| | |
|---|---|
| Ansatzmenge | 400 g |
| Rührerdrehzahl | 1300 U/min |
| Manteltemperatur | 50- 60°C |

- Herstellung erfolgt gemäß Beispiel 19
- Auswertung : Wirkstofffreisetzung

| Zeit in min | 30 | 60 | 120 | 180 | 240 |
|---|---|---|---|---|---|
| Freisetzung in Puffer PH 7,5/2,5%Texapon | 64,67 | 89,83 | 99,98 | 101,78 | 100,99 |

Wirkstofffreisetzung siehe Anlage 19

### Beispiel 23:

### Retigabin- Tabletten

- Rezeptur Schmelzgranulat:

| Substanz | Einwaage |
|---|---|
| Retigabin | 332 g |
| Saccharosestearat S- 1570 | 68 g |

- Parameter:

| | |
|---|---|
| Ansatzmenge | 400 g |
| Rührerdrehzahl | 1300 U/min |
| Manteltemperatur | 50- 60°C |

- Herstellung gemäß Beispiel 19
- Rezeptur Coating:

| Substanz | Einwaage |
|---|---|
| Retigabin- Schmelzgranulat mit 17% Saccharosestearat HLB 15 | 1000 g |
| Eudragit L 30 D- 55 | 400 g (entspricht 120 g Lacktrockensubstanz) |
| Talkum | 60 g |
| Triethylcitrat | 12 g |

Es werden die Schmelzgranulate aus 5 Ansätzen vereinigt und in einem Rotorgranulator bei einer Zuluft von 50°C bei 300 U/min mit einer Suspension aus Eudragit L 30 D-55, Talkum und Triethylcitrat in 536 g gereinigtem Wasser besprüht. Anschließend wird bis zu einer Produkttemperatur von 33°C getrocknet.

Das so gecoatete Granulat wird mit 30 Gew.-% Mikrokristalliner Cellulose und 5 Gew.- % Croscarmellose Natrium 10 min in einer Turbula homogenisiert.

Die Tablettiermischung wird zu Tabletten oblong, 17X8 mm gewölbt mit einer durchschnittlichen Druckfestigkeit von 87 N verpreßt.

| Zeit in min | 15 | 30 | 60 | 120 | 180 | 240 |
|---|---|---|---|---|---|---|
| Freisetzung in % in 0,1 N HCl | 50,3 | 68,8 | 83 | 88,3 | | |
| In Puffer pH 7,5/1,7% Texapon | 16,1 | 30,4 | 55,7 | 83,4 | 95,2 | 99,2 |

Wirkstofffreisetzung in 0,1 N HCl siehe Anlage 20 a
Wirkstofffreisetzung in Puffer pH 7,5, 1,7 % Texapon siehe Anlage 20 b ,

### Beispiel 24:

### "Hot melt coating" von Retigabin- Schmelzgranulat mit 10 % Saccharosestearat HLB- Wert 1

- Rezeptur gecoatetes Schmelzgranulat:

| Substanz | Einwaage |
|---|---|
| Retigabin- Schmelzgranulat (90 Gew.-% Retigabin und 10 Gew.- % | 500 g |
| Saccharosestearat S- 170) | |
| Saccharosestearat S- 170 | 55,6 g |

- Parameter

| | |
|---|---|
| Ansatzmenge | 555,6 g |
| Rührerdrehzahl | 700 U/min |
| Manteltemperatur | 52°C |

In einem Intensivmischer vom Typ GP1 der Fa. Aeromatic- Fielder werden das Retigabin- Schmelzgranulat bei einer Manteltemperatur von 52°C unter Rühren erwärmt. Bei einer Produkttemperatur von 30°C wird Saccharosestearat S- 170 zugegeben und weitere 7 min bei eingeschaltetem Zerhacker (3000 U/min) granuliert. Das gecoatete Granulat wird entnommen und über ein Sieb der Maschenweite 1,4 mm gesiebt.
- Ergebnisse Korngrößenverteilung

| Kornklasse [µm] | Anteil [%] |
|---|---|
| > 1000 | 3,4 |
| > 800 | 4,0 |
| > 500 | 34,4 |
| > 315 | 40,9 |
| > 160 | 14,0 |
| > 50 | 3,4 |
| < 50 | 0 |

| Zeit in min | 15 | 30 | 60 | 120 | 180 | 240 | 360 | 480 |
|---|---|---|---|---|---|---|---|---|
| Freisetzung in % in 0,1 N HCl | 23,8 | 43,6 | 71,4 | 94,4 | | | | |
| In Puffer pH 7,5/2,5% Texapon | | 7,2 | 13,2 | 19,8 | 25,4 | 31 | 40,2 | 47,4 |

Wirkstofffreisetzung in 0,1 N HCl siehe Anlage 21 a
Wirkstofffreisetzung in Puffer pH 7,5, 2,5 % Texapon siehe Anlage 21b

### Beispiel 25:

### Dexlipotam (Tromethamolsalz der R⁺- Thioctsäure) mit 22,7% Saccharosestearat HLB-Wert 15

- Rezeptur:

| Substanz | Einwaage |
|---|---|
| Dexlipotam | 255g |
| Saccharosestearat S-170 | 75g |

- Parameter:

| | | |
|---|---|---|
| Ansatzmenge | 330 g | |
| Rührerdrehzahl | 500 U/min | |
| Zerhackerdrehzahl | 3000 U/min | |
| Manteltemperatur | 55,0- 80,0°C | |

- Herstellung erfolgt gemäß Beispiel 1.
- Auswertung: Wirkstofffreisetzung

| Zeit in min | 15 | 30 | 45 |
|---|---|---|---|
| Freisetzung in % in Puffer pH 4,5 | 93 | 95 | 101 |

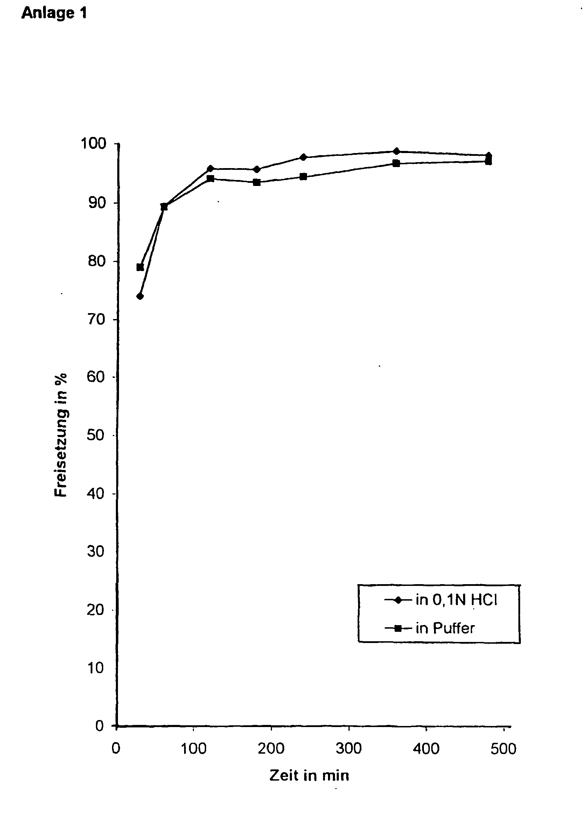

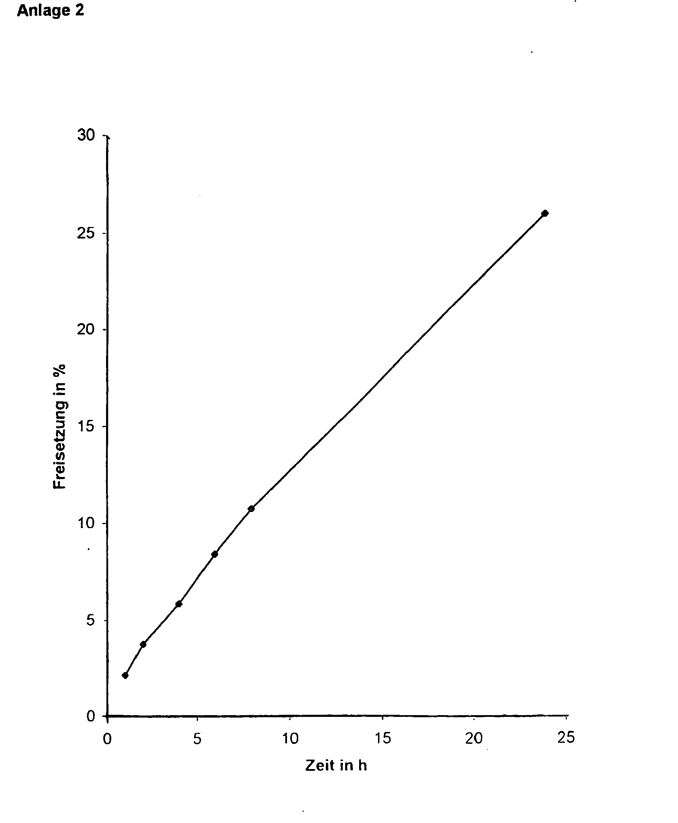

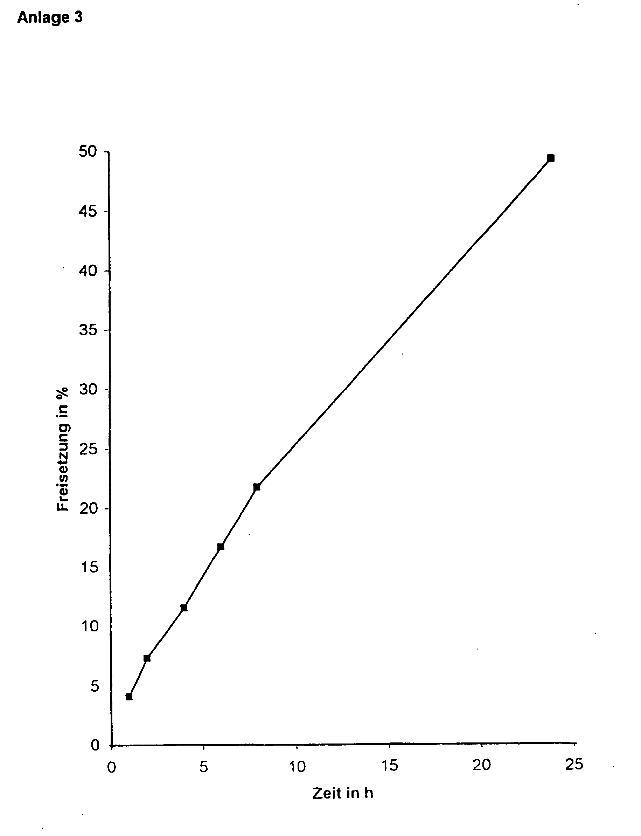

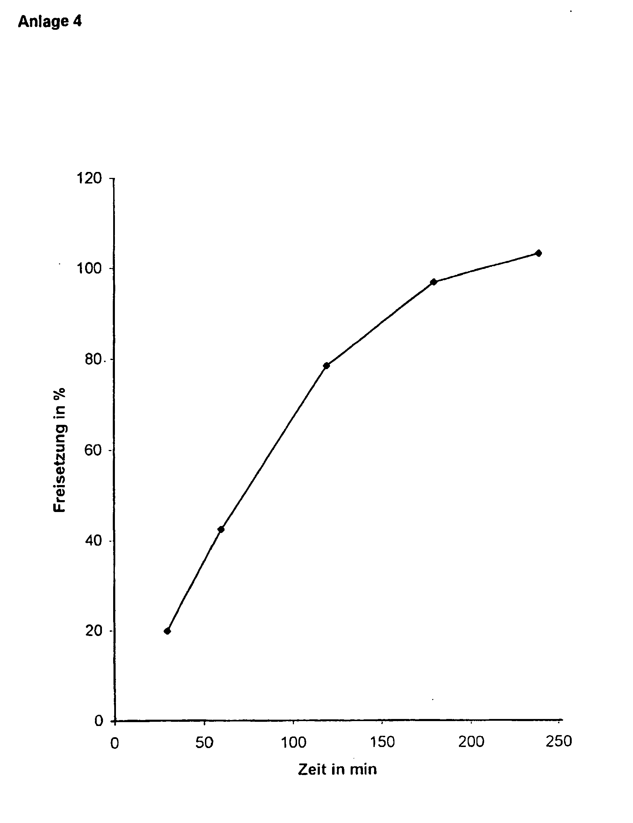

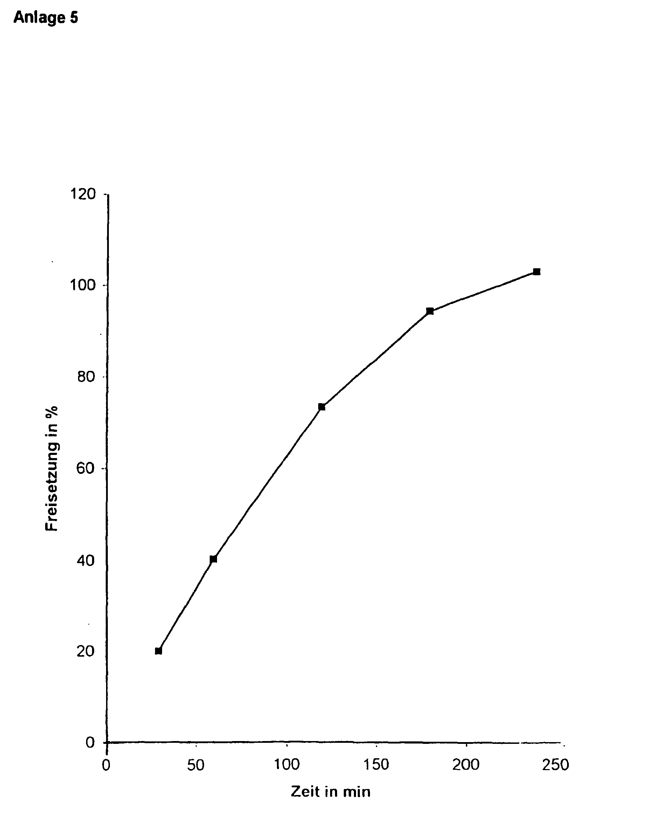

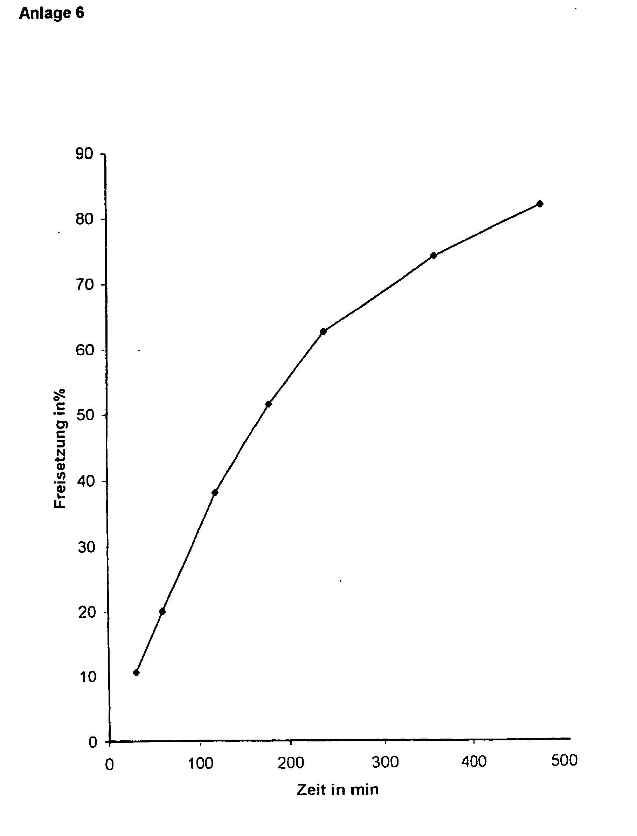

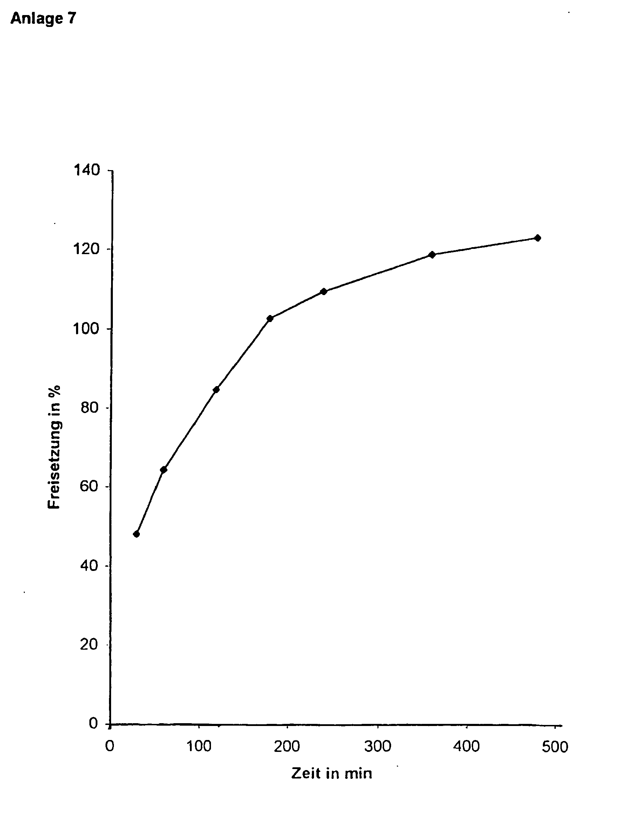

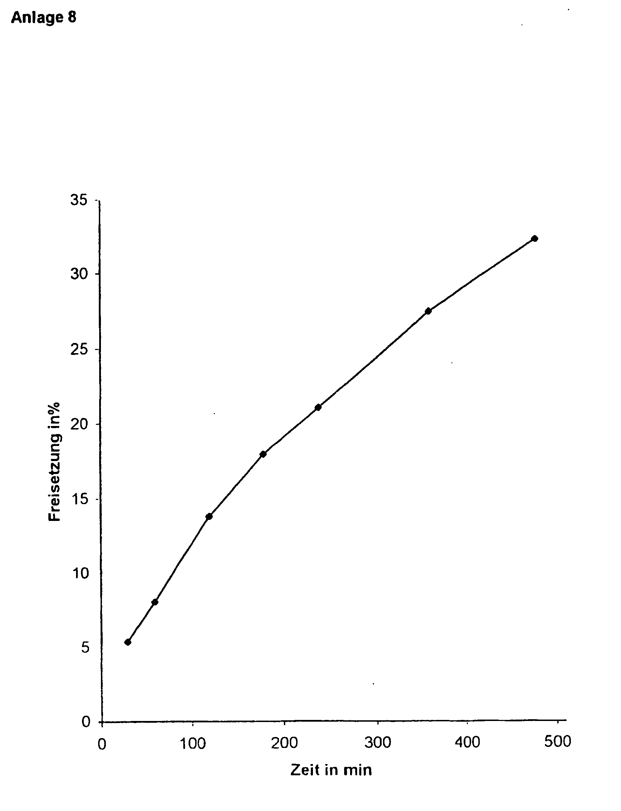

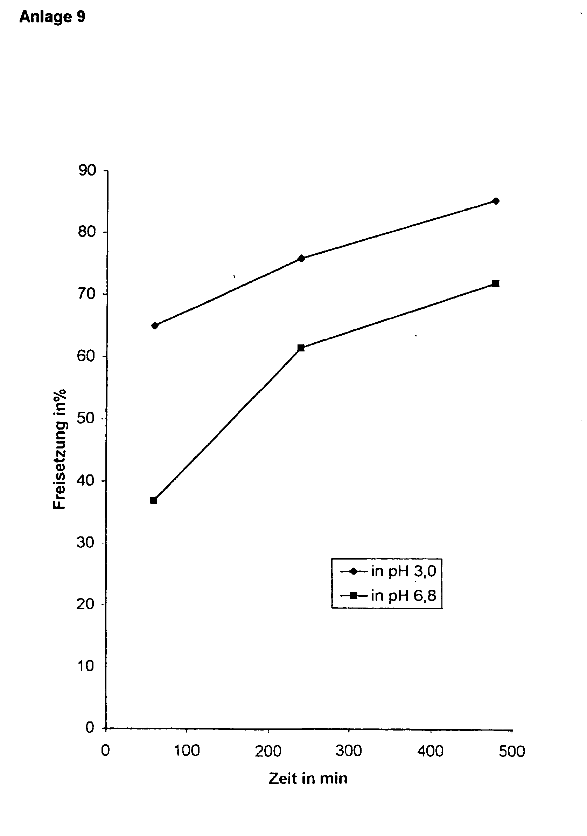

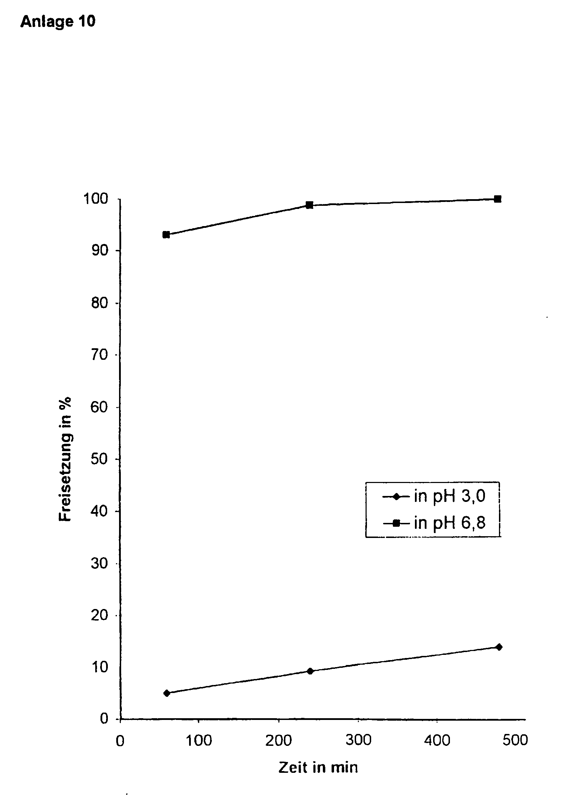

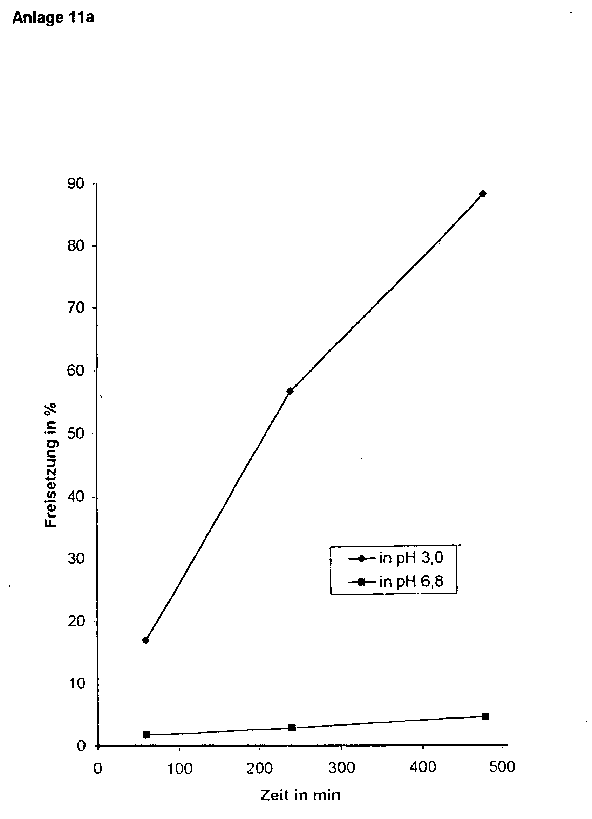

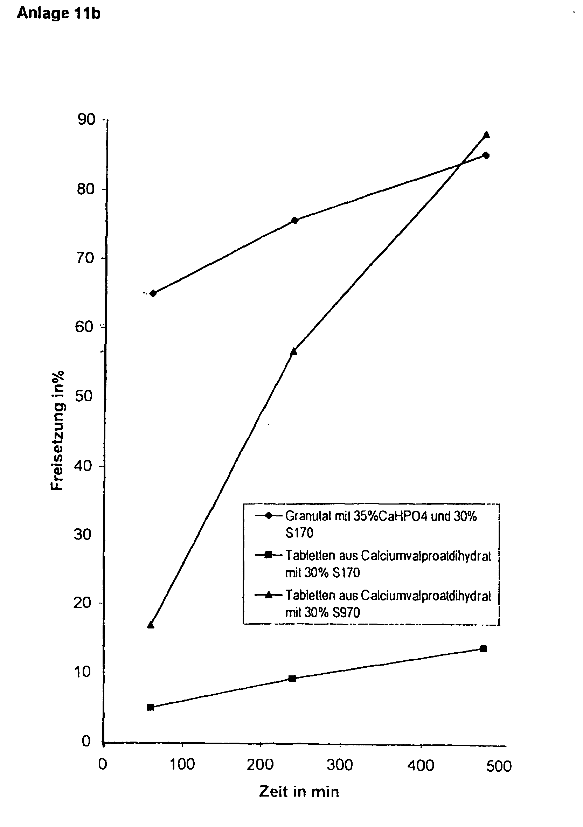

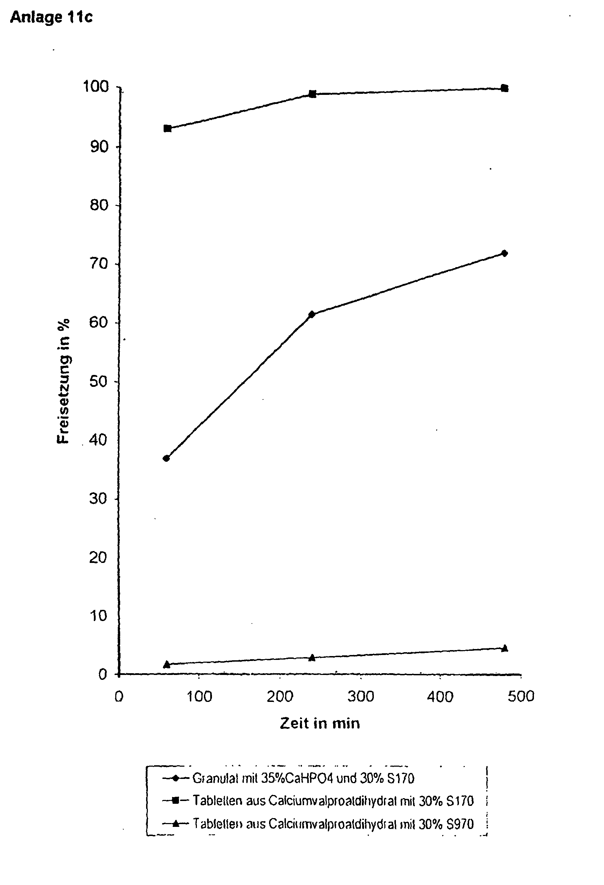

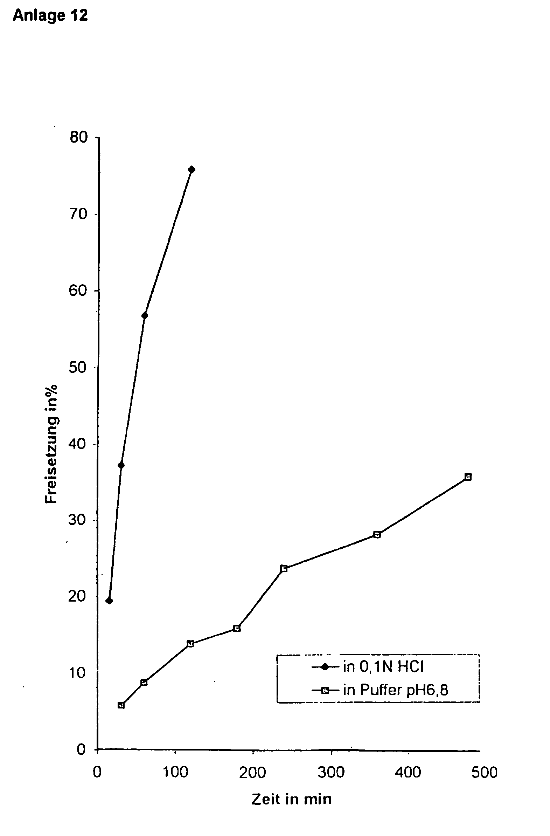

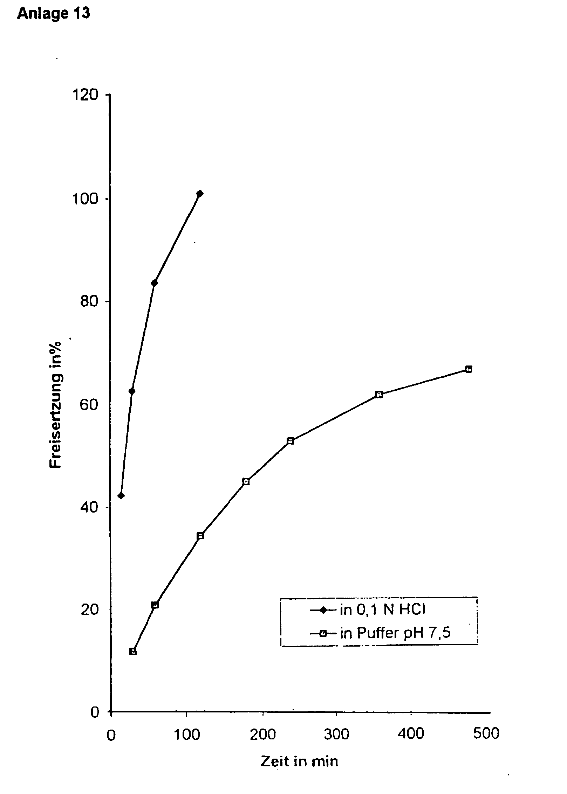

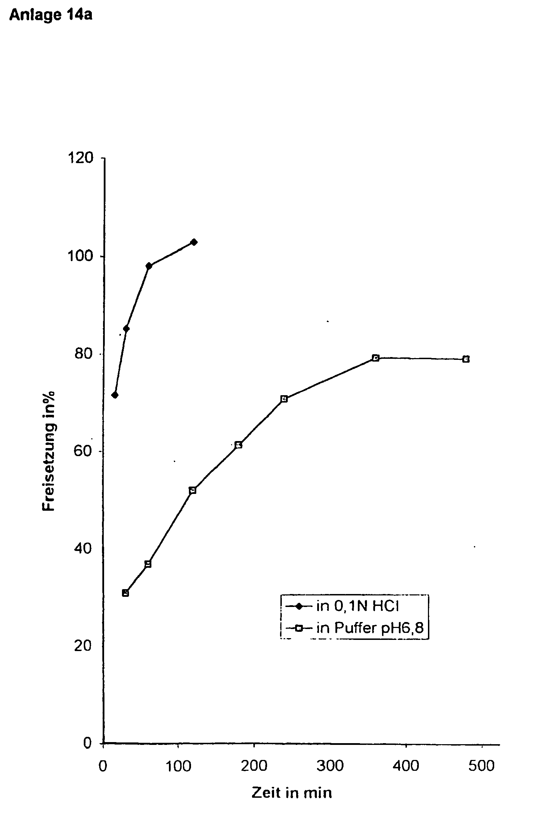

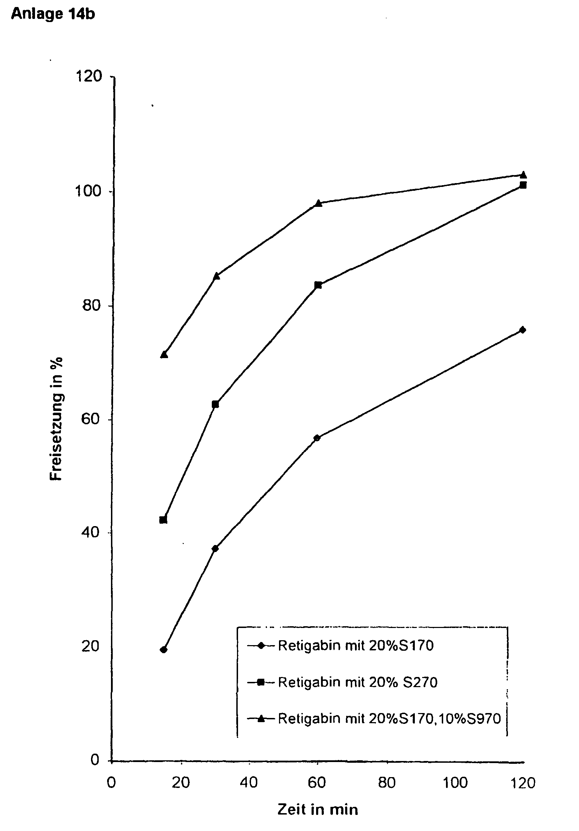

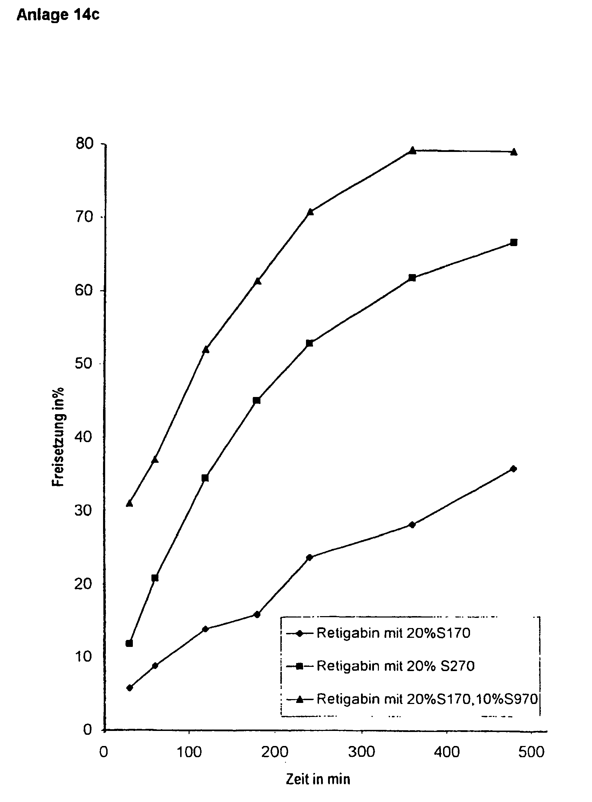

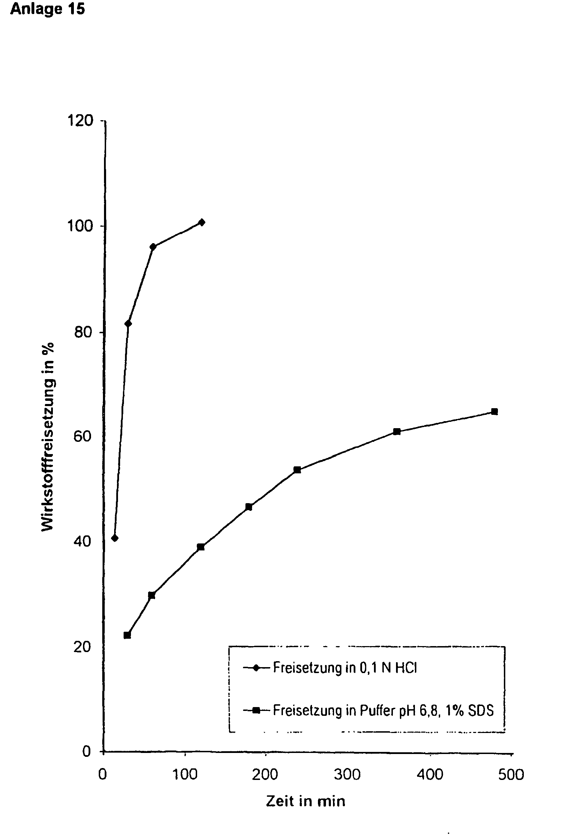

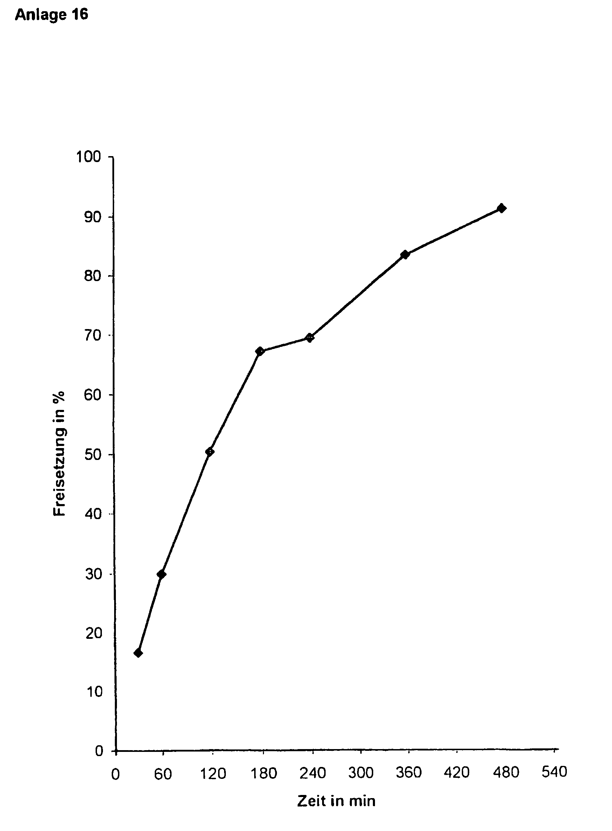

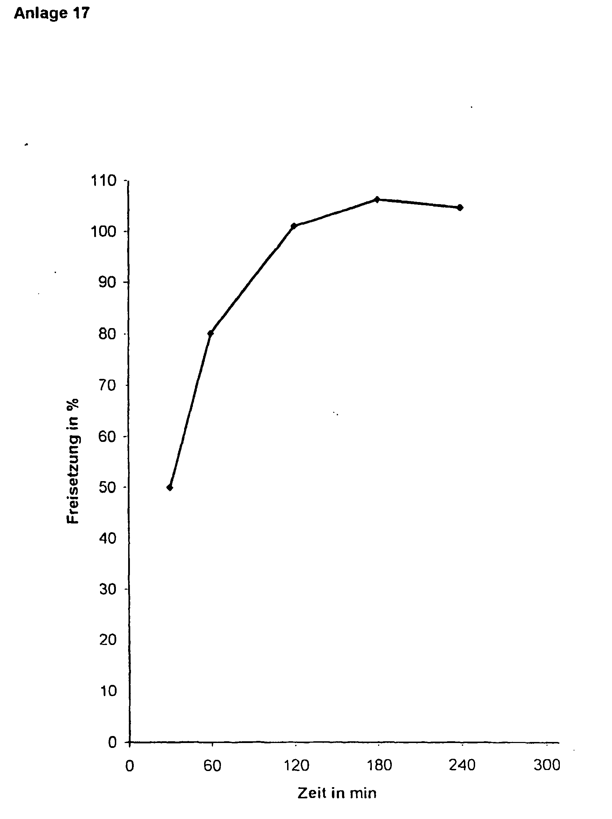

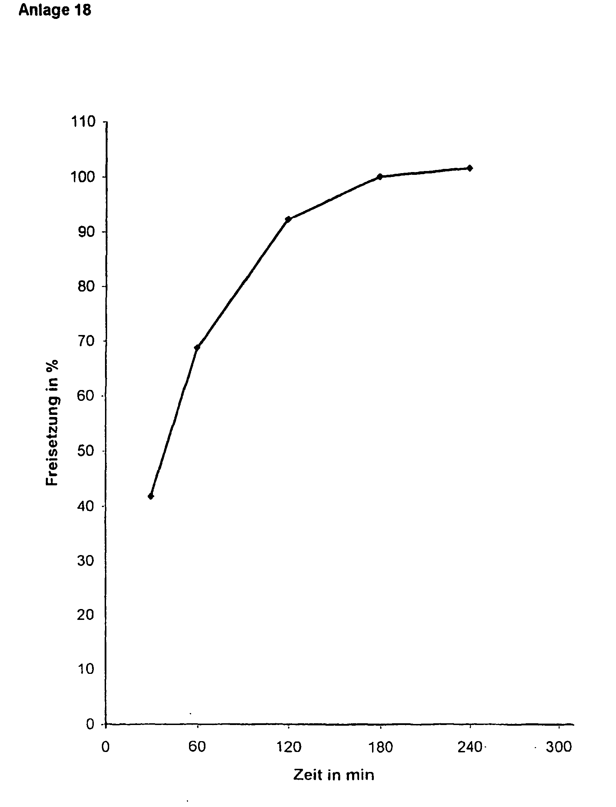

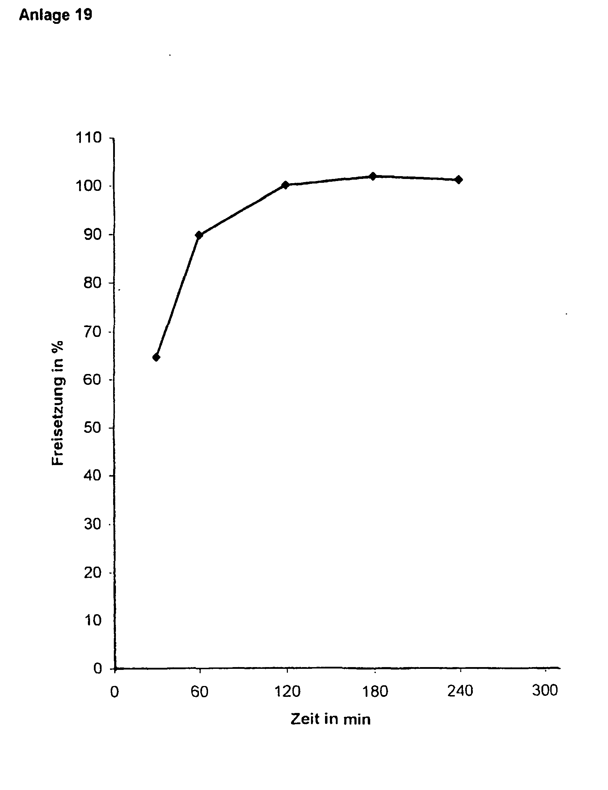

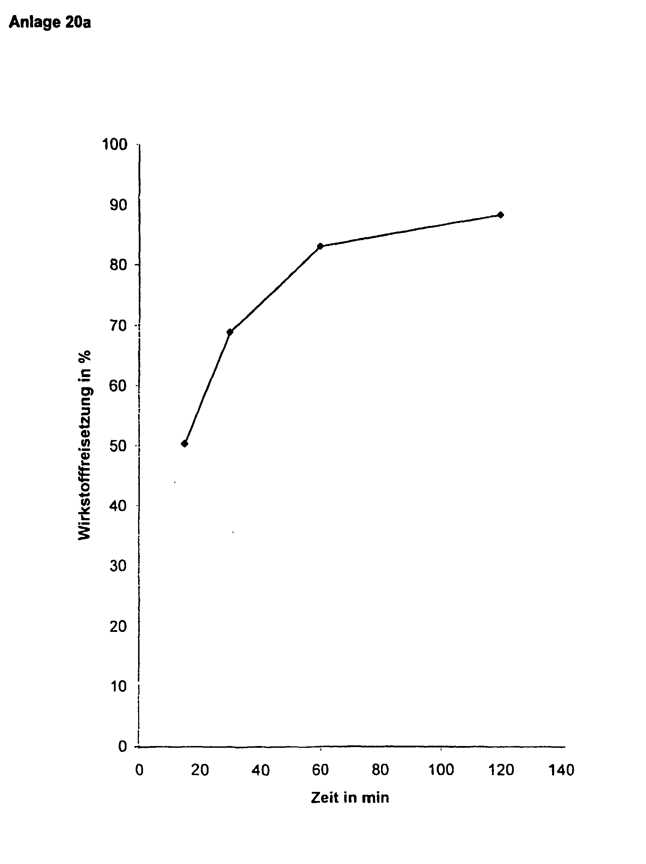

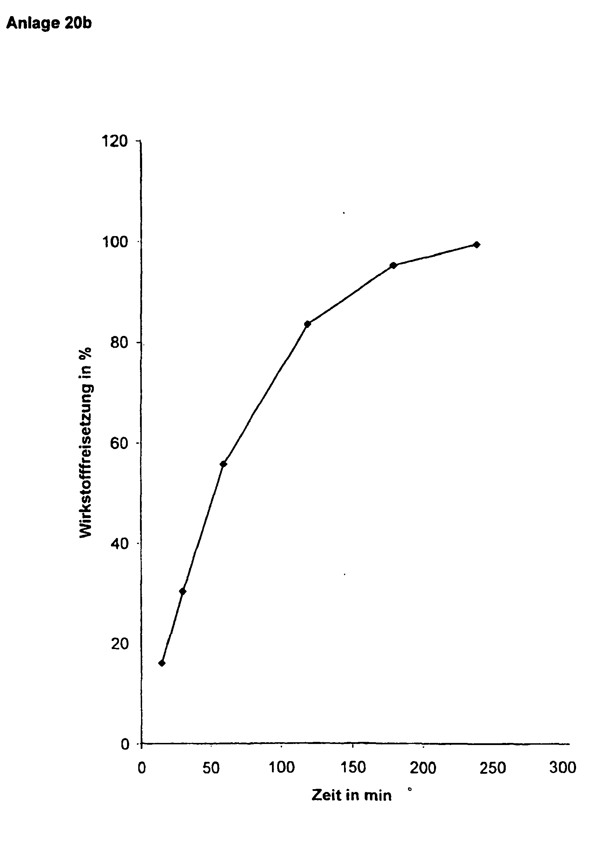

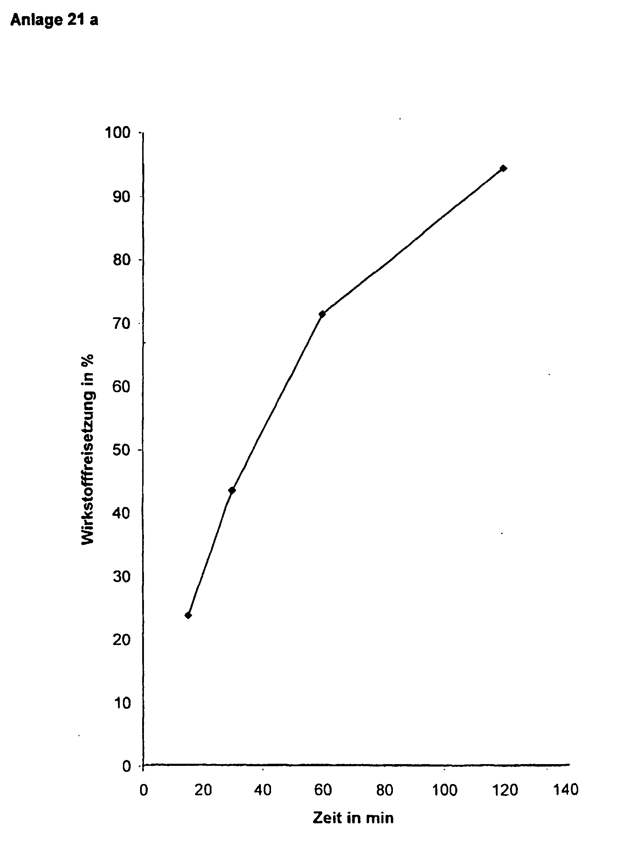

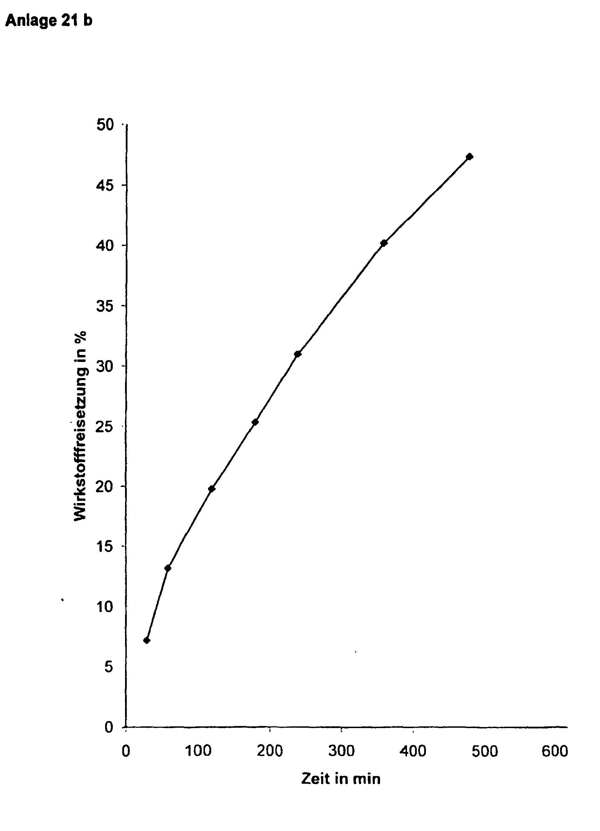

## Patentansprüche

1. Orale pharmazeutische Formulierungen mit variabel einstellbarem Freisetzungsverhalten umfassend einen zu granulierenden Anteil als Innenphase, welcher gegebenenfalls mit Saccharosefettsäureester umhüllt ist, **dadurch gekennzeichnet,**
- **dass** die Innenphase aus einem oder mehreren Wirkstoffen, einem oder mehreren Saccharosefettsäureestern als alleinigem freisetzungssteuernden Mittel und gegebenenfalls einem oder mehreren pharmazeutisch gebräuchlichen Hilfsstoffen besteht und
- **dass** die Innen phase durch gemeinsame Schmelzgranulation oder Schmelzpelletierung der Komponenten erhältlich ist, wobei die Ausgangsstoffe unter Rühren oder in der Wirbelschicht auf eine Temperatur erwärmt werden, bei der die verwendeten Saccharosefettsäureester oberflächlich erweichen oder anschmelzen oder ihre Schmelztemperatur erreicht wird, und nach erfolgter Granulatbildung gekühlt werden,
- wobei Restlösungsmittel im Produkt entfallen.

2. Pharmazeutische Formulierungen nach Anspruch 1 **dadurch gekennzeichnet, dass** es sich um "Single unit" oder "Multiple units" Arzneiformen handelt.

3. Pharmazeutische Formulierungen nach Anspruch 1 oder 2 in Form von oralen Darreichungsformen ausgewählt aus Granulaten, Pellets, Tabletten, Filmtabletten, Mikrotabletten, Dragees, Kapseln oder spezielle therapeutische Systeme.

4. Pharmazeutische Formulierungen nach Anspruch 1 **dadurch gekennzeichnet, dass** die eingesetzten Saccharosefettsäureester bestehen aus Mono-, Di,-Tri- oder Polyestern der Saccharose mit mittel- bis langkettigen gesättigten und/oder ungesättigten Fettsäuren.

5. Pharmazeutische Formulierungen nach Anspruch 4 **dadurch gekennzeichnet, dass** als Saccharosefettsäureester Ester der Saccharose mit C12-C22-Fettsäuren eingesetzt werden.

6. Pharmazeutische Formulierungen nach Anspruch 1 oder 5 **dadurch gekennzeichnet, dass** der HLB-Wert der eingesetzten Saccharosefettsäureester 1 bis 16 beträgt.

7. Pharmazeutische Formulierungen nach Anspruch 1 oder 5 **dadurch gekennzeichnet, dass** die eingesetzten Saccharosefettsäureester ihren Schmelzpunkt oder Schmelzbereich im Temperaturbereich von 30 bis 200 °C haben.

8. Pharmazeutische Formulierungen nach Anspruch 7 **dadurch gekennzeichnet, dass** die eingesetzten Saccharosefettsäureester ihren Schmelzpunkt oder Schmelzbereich im Temperaturbereich von 40 bis 150 °C haben.

9. Pharmazeutische Formulierungen nach Anspruch 1 **dadurch gekennzeichnet, dass** Saccharosefettsäureester mit einem Anteil von 1 - 60 Gew.-% in der Umhüllung bezogen auf die umhüllte Arzneiform enthalten sind.

10. Pharmazeutische Formulierungen nach Anspruch 9 **dadurch gekennzeichnet, dass** Saccharosefettsäureester mit einem Anteil von 3 - 20 Gew.-% in der Umhüllung bezogen auf die umhüllte Arzneiform enthalten sind.

11. Pharmazeutische Formulierungen nach Anspruch 1 **dadurch gekennzeichnet, dass** als Wirkstoffe gut wasserlösliche bis praktisch wasserunlösliche Wirkstoffe eingesetzt werden.

12. Pharmazeutische Formulierungen nach Anspruch 1 oder 11 **dadurch gekennzeichnet, dass** Wirkstoffe ausgewählt aus den Indikationsgebieten Analeptika/Antihypoxämika, Analgetika/Antirheumatika, Antiallergika, Antiarrhytmika, Antidementiva, Antidiabetika, Antiemetika/Antivertiginosa, Antiepileptika, Antihypertonika, Antihypotonika, Broncholytika, Antiasthmatika, Diuretika, durchblutungsfördernde Mittel, Hypnotika/Sedativa, Koronarmittel, Lipidsenker, Migränemittel, Muskelrelaxantia, Parkinsonmittel und Psychopharmaka eingesetzt werden.

13. Pharmazeutische Formulierungen nach Anspruch 1 oder 11 enthaltend einen oder mehrere Wirkstoffe ausgewählt aus Coffein, Diclofenac, Morphin, Tramadol, Tilidin, Flupirtin, Azelastin, Pseudoephedrin, Chinidin, Disopyramid, Diltiazem, Verapamil, Piracetam, Nicergolin, Xantinonicotinat, Pentifyllin, Vincamin, Glibenclamid, Betahistindimesilat, Dimenhydrinat, Carbamazepin, Valproinsäure, Calciumvalproatdihydrat, Retigabin, Talinolol, Fosinopril, Doxazosin, Metoprolol, Nifedipin, Norfenefrin-HCl, Dihydro ergotaminmesilat, Salbutamol, Terbutalinsulfat, Theophyllin, Furosemid, Piretanid, Buflomedil, Naftidrofuryl, Pentoxifyllin, Glyceroltrinitrat, Isosorbidmononitrat, Isosorbiddinitrat, Molsidomin, Bezafibrat, Fenofibrat, Xantinol, Sumatriptan, Levodopa, Benserazid, Carbidopa, Amitriptylin HCl, Venlafaxin-HCl, Thioridazin-HCl, Lithiumcarbonat, Lithiumacetat, Thioctsäure oder R-Thioctsäure und ihre Salze, wie Dexlipotam.

14. Pharmazeutische Formulierungen nach Anspruch 1 oder 11 enthaltend Flupirtin, Tramadol, Nifedipin, Carbamazepin, Calciumvalproat oder Retigabin.

15. Verfahren zur Herstellung pharmazeutischer Formulierungen gemäss Anspruch 1 durch Schmelzgranulation oder Schmelzpelletierung, **dadurch gekennzeichnet, dass** in einem geeigneten Gerät die Ausgangsstoffe unter Rühren oder in der Wirbelschicht auf eine Temperatur, bei der der oder die verwendeten Saccharosefettsäureester erweichen, oberflächlich anschmelzen oder ihre Schmelztemperatur erreicht ist, erwärmt und nach erfolgter Granulatbildung gekühlt werden.

16. Verfahren zur Herstellung pharmazeutischer Formulierungen nach Anspruch 15 **dadurch gekennzeichnet, dass** in einem geeigneten Gerät der oder die geschmolzenen Saccharosefettsäureester zum erwärmten Wirkstoffpulver gegeben werden.

17. Verfahren zur Herstellung pharmazeutischer Formulierungen nach Anspruch 15 oder 16 **dadurch gekennzeichnet, dass** als geeignetes Gerät ein schnelllaufender Mischer, ein Wirbelschichtgerät oder ein Rotorgranulator verwendet wird.

18. Verfahren zur Herstellung pharmazeutischer Formulierungen gemäss Anspruch 1, **dadurch gekennzeichnet, dass** die nach dem Verfahren gemäß Anspruch 15 hergestellten Granulate oder Pellets durch das "hot melt coating"-Verfahren oder "powder coating"-Verfahren umhüllt werden.

19. Verfahren zur Herstellung pharmazeutischer Formulierungen nach Anspruch 18 **dadurch gekennzeichnet, dass** beim "hot melt coating" und beim "powder coating" mit dem oder den Saccharosefettsäureester(n) allein oder in Kombination mit Weichmachern gearbeitet wird.

20. Verfahren zur Herstellung pharmazeutischer Formulierungen nach Anspruch 19 **dadurch gekennzeichnet, dass** als Weichmacher Triethylcitrat, Acetyltriethylcitrat, Triacetin oder Dibutylsebacat verwendet werden.

## Claims

1. Oral pharmaceutical formulations with variably settable releasing behaviour comprising a proportion to be granulated as internal phase, which is possibly coated by sucrose fatty acid ester, **characterized by** that
- the internal phase consists of one or several active agent(s), one or several sucrose fatty acid ester(s) as the agent(s) alone controlling the releasing behaviour and possibly one or several pharmaceutically usual auxiliary agent(s), and
- the internal phase is available by common melt granulation or melt pelletizing of the components, whereby the starting substances are heated with agitation or in a fluidized bed to a temperature at which the used sucrose fatty acid esters soften or begin to melt at the surface or their melting temperature is reached, and are cooled after granulating.

2. Pharmaceutical formulations to claim 1 **characterized by** that the pharmaceutical dosage forms are "single units" or "multiple units".

3. Pharmaceutical formulations to claim 1 or 2 in oral administration forms chosen from granulates, pellets, tablets, film tablets, micro tablets, dragees, capsules or special therapeutical systems.

4. Pharmaceutical formulations to claim 1 **characterized by** that the used sucrose fatty acid esters consist of monoesters, diesters, triesters or polyesters of the sucrose with medium- to long-chain saturated and/or unsaturated fatty acids.

5. Pharmaceutical formulations to claim 4 **characterized by** that as sucrose fatty acid esters esters of the sucrose with C12-C22 fatty acids are used.

6. Pharmaceutical formulations to claim 1 or 5 **characterized by** that the HLB of the used sucrose fatty acid esters is 1 to 16.

7. Pharmaceutical formulations to claim 1 or 5 **characterized by** that the melting point or melting range of the used sucrose fatty acid esters is in the temperature range of between 30 to 200 °C.

8. Pharmaceutical formulations to claim 7 **characterized by** that the melting point or melting range of the used sucrose fatty acid esters is in the temperature range of between 40 to 150°C.

9. Pharmaceutical formulations to claim 1 **characterized by** that sucrose fatty acid esters are contained in the coating with a proportion of 1 to 60 wt.-% in relation to the coated pharmaceutical form.

10. Pharmaceutical formulations to claim 9 **characterized by** that sucrose fatty acid esters are contained in the coating with a proportion of 3 to 20 wt.-% in relation to the coated pharmaceutical form.

11. Pharmaceutical formulations to claim 1 **characterized by** that agents are used as active agents that are readily water-soluble to practically water-insoluble.

12. Pharmaceutical formulations to claim 1 or 11 **characterized by** that active agents are used chosen from the indication fields of analeptics/antihypoxaemics, analgesics/antirheumatics, antiallergics, antiarrhythmics, antidementive drugs, antidiabetics, antiemetics/antivertiginose drugs, antiepileptics, antihypertensive drugs, hypertensors, broncholytics, antiasthmatics, diuretics, blood flow stimulating drugs, hypnotics/sedatives, coronary drugs, lipid lowering drugs, migraine drugs, muscle relaxants, Parkinson drugs and psychiatric drugs.

13. Pharmaceutical formulations to claim 1 or 11 containing one or several agents chosen from caffeine, diclofenac, morphine, tramadol, tilidine, flupirtine, azelastine, pseudoephedrine, chinidine, diisopyramide, diltiazem, verapamil, piracetam, nicergoline, xantinol nicotinate, pentifylline, vincamine, glibenclamide, betahistine dimesilate, dimenhydrinate, carbamazepine, valproine acid, calcium valproate dihydrate, retigabine, talinolol, fosinopril, doxazosine, metoprolol, nifedipine, norfenefrine HCl, dihydroergotamin mesilate, salbutamol, terbutaline sulfate, theophylline, furosemide, piretanide, buflomedil, naftidrofuryl, pentoxifylline, glycerol trinitrate, isosorbide mononitrate, isosorbide dinitrate, molsidomine, bezafibrate, fenofibrate, xantinol, sumatriptane, levodopa, benserazide, carbidopa, amitriptyline HCl, venlafaxine HCl, thioridazine HCl, lithium carbonate, lithium acetate, thioctic acid oder R-thioctic acid and their salts such as dexlipotam.

14. Pharmaceutical formulations to claim 1 or 11 containing flupirtine, tramadol, nifedipine, carbamazepine, calcium valproate or retigabine.

15. Process for the manufacture of pharmaceutical formulations to claim 1 by melt granulation or melt pelletizing **characterized by** that in a suitable apparatus the starting substances are heated with agitation or in a fluidized bed to a temperature at which the used sucrose fatty acid ester(s) soften, begin to melt at the surface or their melting temperature is reached, and are cooled after granulating.

16. Process for the manufacture of pharmaceutical formulations to claim 15 **characterized by** that in a suitable apparatus the melted sucrose fatty acid ester(s) are added to the heated active agent powder.

17. Process for the manufacture of pharmaceutical formulations to claim 15 or 16 **characterized by** that as a suitable apparatus a high-speed mixer, a fluidized bed apparatus or a rotary granulator is used.

18. Process for the manufacture of pharmaceutical formulations to claim 1 **characterized by** that the granulates or pellets made by the process to claim 15 are coated by the "hot melt coating" process or the "powder coating" process.

19. Process for the manufacture of pharmaceutical formulations to claim 18 **characterized by** that in "hot melt coating" and "powder coating" the sucrose fatty acid ester(s) alone or in combination with softeners are used.

20. Process for the manufacture of pharmaceutical formulations to claim 19 **characterized by** that as softeners triethyl citrate, acetyl triethyl citrate, triacetine or dibutyl sebacate are used.

## Revendications

1. Formulations pharmaceutiques orales présentant des comportements de libération pouvant être ajustés de façon variable, comprenant une partie à granuler comme phase interne qui est éventuellement enrobée ou enveloppée par des esters d'acides gras de saccharose, **caractérisées en ce que**
- la phase interne est constituée d'un ou de plusieurs principes actifs, d'un
ou de plusieurs esters d'acides gras de saccharose comme moyen unique de commande de libération et éventuellement, d'un ou des plusieurs adjuvants usuels d'un point de vue pharmaceutique,
- la phase interne est obtenue par granulation par fusion ou pastillage par fusion des composantes, les substances de départ étant portées, par agitation ou dans un lit fluidisé, à une température à laquelle les esters d'acides gras de saccharose utilisés ramollissent ou commencent à fondre en surface, ou à laquelle leur température de fusion est atteinte, et en refroidissant après la formation de granulat.

2. Formulations pharmaceutiques selon la revendication 1, **caractérisées en ce qu'**il s'agit de formes galéniques en unités simples ou en unités multiples.

3. Formulations pharmaceutiques selon la revendication 1 ou 2, se présentant sous une forme galénique orale choisie parmi les granulats, les pastilles, les comprimés, les comprimés pelliculés, les micro-comprimés, les dragées, les gélules ou des systèmes thérapeutiques spécifiques.

4. Formulations pharmaceutiques selon la revendication 1, **caractérisées en ce que** les esters d'acides gras de saccharose utilisés comprennent les mono-, di-, tri- ou polyesters de saccharose avec des acides gras saturés et/ou insaturés à chaîne moyenne ou longue.

5. Formulations pharmaceutiques selon la revendication 4, **caractérisées en ce que** l'on utilise comme esters d'acides gras de saccharose, des esters de saccharose avec des acides gras en C12 à C22.

6. Formulations pharmaceutiques selon la revendication 1 ou 5, **caractérisées en ce que** la valeur HLB des esters d'acides gras de saccharose utilisés est de 1 à 16.

7. Formulations pharmaceutiques selon la revendication 1 ou 5, **caractérisées en ce que** la plage de température du point de fusion ou de l'intervalle de fusion des esters d'acides gras de saccharose utilisés est de 30 à 200° C.

8. Formulations pharmaceutiques selon la revendication 7, **caractérisées en ce que** la plage de température du point de fusion ou de l'intervalle de fusion des esters d'acides gras de saccharose utilisés est de 40 à 150° C.

9. Formulations pharmaceutiques selon la revendication 1, **caractérisées en ce que** les esters d'acides gras de saccharose sont contenus en une proportion de 1 à 60 % en poids dans l'enrobage, par rapport à la forme galénique enrobée.

10. Formulations pharmaceutiques selon la revendication 9, **caractérisées en ce que** les esters d'acides gras et de saccharose sont contenus en une proportion de 3 à 20 % en poids dans l'enrobage, par rapport à la forme galénique enrobée.

11. Formulations pharmaceutiques selon la revendication 1, **caractérisées en ce que** l'on utilise comme principes actifs, des principes actifs bien hydrosolubles à pratiquement insolubles dans l'eau.

12. Formulations pharmaceutiques selon la revendication 1 ou 11, **caractérisées en ce que** les principes actifs sont choisis dans les domaines d'indication: analeptiques/anti-hypoxémiques, analgésiques/antirhumatismaux, anti-allergiques, anti-arythmiques, anti-démentiels, anti-diabétiques, anti-émétiques/anti-vertigineux, anti-épileptiques, anti-hypertensifs, anti-hypotensifs, broncho-dilatateurs, anti-asthmatiques, diurétiques, agents favorisant l'irrigation par le sang, hypnotiques/sédatifs, agents coronariens, hypolipidémiants, anti-migraineux, myorelaxants, anti-parkinsoniens et agents psychotropes.

13. Formulations pharmaceutiques selon la revendication 1 ou 11, contenant un ou plusieurs principes actifs choisis parmi : la caféine, le diclofénac, la morphine, le tramadol, la tilidine, la flupirtine, l'azélastine, la pseudo-éphédrine, la chinidine, le disopyramide, la diltiazem, le vérapamil, le piracétam, la nicergoline, le xantinonicotinate, la pentifylline, la vincamine, le glibenclamide, le dimésilate de bêta-histine, le dimenhydrinate, la carbamazépine, l'acide valproïque, le dihydrate de valproate calcique, la rétigabine, le talinolol, le fosinopril, la doxazosine, le métoprolol, la nifédipine, le norfénéfrine-HCl, le mésilate de dihydro-ergotamine, le salbutamol, le sulfate de terbutaline, la théophylline, le furosémide, le pirétanide, le buflomédil, le naftidrofuryle, la pentoxifylline, le trinitrate de glycérol, le mononitrate d'isosorbide, le dinitrate d'isosorbide, la molsidomine, le bézafibrate, le fénofibrate, le xantinol, le sumatriptan, la lévodopa, le bensérazide, la carbidopa, l'amitriptyline HCl, le venlafaxine-HCl, le thioridazine-HCl, le carbonate de lithium, l'acétate de lithium, l'acide thioctique, ou l'acide R-thioctique et leurs sels, tels que le dexlipotam.

14. Formulations pharmaceutiques selon la revendication 1 ou 11, contenant de la flupirtine, du tramadol, de la nifédipine, de la carbamazépine, du valproate de calcium ou de la rétigabine.

15. Procédé de production des formulations pharmaceutiques selon la revendication 1 par granulation par fusion ou pastillage par fusion, **caractérisé en ce que**, dans un appareil approprié, les substances de départ sont portées à une température à laquelle, par agitation ou dans un lit fluidisé, les esters d'acides gras de saccharose utilisés ramollissent ou fondent en surface ou à laquelle leur température de fusion est atteinte, et en refroidissant après la formation du granulat.

16. Procédé de production de formulations pharmaceutiques selon la revendication 15, **caractérisé en ce que** l'on ajoute à la poudre de principe actif chauffée dans un appareil approprié, le ou les esters d'acides gras de saccharose fondus.

17. Procédé de production de formulations pharmaceutiques selon la revendication 15 ou 16, **caractérisé en ce que** l'on utilise comme appareil approprié, un mélangeur rapide, un appareil à lit fluidisé ou un granulateur rotatif.

18. Procédé de production des formulations pharmaceutiques selon la revendication 1, **caractérisé en ce que** les granulats ou pastilles produits d'après le procédé selon la revendication 15 sont enrobés par le procédé de revêtement par fusion "hot melt coating" ou d'enrobage en poudre "powder coating".

19. Procédé de production de formulations pharmaceutiques selon la revendication 18, **caractérisé en ce que** l'on utilise, dans le procédé "hot melt coating" ou dans le procédé "powder coating", le ou les ester(s) d'acides gras de saccharose seul(s) ou en combinaison avec des plastifiants.

20. Procédé de production de formulations pharmaceutiques selon la revendication 19, **caractérisé en ce que** l'on utilise comme plastifiants, du triéthylcitrate, de l'acétyltriéthylcitrate, de la triacétine ou du dibutyl-sébaçate.
